(19) Europäisches Patentamt
European Patent Office
Office européen des brevets

(11) EP 3 895 686 B1

(12) **EUROPEAN PATENT SPECIFICATION**

(45) Date of publication and mention of the grant of the patent:
**09.04.2025 Bulletin 2025/15**

(21) Application number: **20169365.2**

(22) Date of filing: **14.04.2020**

(51) International Patent Classification (IPC):
***A61K 8/36*** (2006.01) ***A61K 8/365*** (2006.01)
***A61K 8/49*** (2006.01) ***A61K 8/9794*** (2017.01)
***A61Q 7/00*** (2006.01)

(52) Cooperative Patent Classification (CPC):
**A61K 8/365; A61K 8/361; A61K 8/498;**
**A61K 8/9794; A61Q 7/00**

(54) **NATURAL COMPOSITION FOR HAIR GROWTH STIMULATION AND HAIR LOSS PREVENTION AND TREATMENT**

NATÜRLICHE ZUSAMMENSETZUNG ZUR STIMULIERUNG DES HAARWUCHSES UND ZUR VORBEUGUNG UND BEHANDLUNG VON HAARAUSFALL

COMPOSITION NATURELLE POUR LA STIMULATION DE LA CROISSANCE DES CHEVEUX ET LA PRÉVENTION ET LE TRAITEMENT DE LA CHUTE DES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.10.2021 Bulletin 2021/42**

(73) Proprietor: **Fraunhofer-Gesellschaft zur Förderung der angewandten Forschung e.V.**
**80686 München (DE)**

(72) Inventor: **STORSBERG, Joachim**
**14476 Potsdam (DE)**

(74) Representative: **Maiwald GmbH**
**Elisenhof**
**Elisenstraße 3**
**80335 München (DE)**

(56) References cited:
**WO-A1-2017/178250**
**WO-A1-2019/005139**
**JP-A- 2006 273 754**
**JP-A- S59 152 307**
**US-A- 5 827 510**

- **FONG PEDRO ET AL: "In silicoprediction of prostaglandin D2 synthase inhibitors from herbal constituents for the treatment of hair loss", JOURNAL OF ETHNOPHARMACOLOGY, ELSEVIER IRELAND LTD, IE, vol. 175, 9 October 2015 (2015-10-09), pages 470 - 480, XP029331834, ISSN: 0378-8741, DOI: 10.1016/J.JEP.2015.10.005**
- **SEMCHIN MUNKHBAYAR ET AL: "Role of Arachidonic Acid in Promoting Hair Growth", ANNALS OF DERMATOLOGY, vol. 28, no. 1, 1 January 2016 (2016-01-01), KR, pages 55, XP055716806, ISSN: 1013-9087, DOI: 10.5021/ad.2016.28.1.55**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 895 686 B1

## Description

Present invention relates to the field of hair loss treatment of any cause. In particular, the invention relates to a composition of ricinoleic acid and arachidonate or ricinoleic acid and diosgenin or ricinoleic acid, arachidonate and diosgenin, preferably in topical formulations for cosmetic or medical treatments for hair growth.

## BACKGROUND ART

The formation of new hair is not a constant process. In each hair follicle, the phases of growth, rest and new development alternate repeatedly. Moreover, the hair growth cycle of each hair is individual and cannot be generalized.

Hair loss due to hair change is a physiological process, as all hairs and hair types have a limited lifespan. A distinction is made between three phases:

1) Anagen phase: The "papilla hair" is firmly attached to the papilla.
The anagen phase, in which the eyelashes grow, takes about 1 to 2 months. The duration of the anagen phase, therefore, influences the length of the eyelashes.
2) Catagen phase: The hair stops growing, detaches from the papilla and moves upwards in the follicle. The hair bulb takes on a bulb shape (bulb hair).
The catagen phase is a transitional phase that lasts for about 15 days for eyelashes.
3) Telogen phase: The hair remains at the level of the sebaceous gland until it is expelled by a hair that is pushing it out. The longest phase of the normal eyelash change is the telogen phase. It is the resting phase and lasts 4 to 9 months.

The cycle of a normal change of eyelashes lasts from 5 to 11 months.

There is an immense interest in the development of effective cosmetic and/ or medical treatments, both to prevent hair loss and to stimulate regrowth of lost hair.

From this viewpoint, a large number of compositions comprising very diverse active ingredients, such as, for example, 2,4-diamino-6-piperidinopyrimidine 3-oxide or "minoxidil", disclosed in U.S. Pat. Nos. 4,139,619 and 4,596,812, or its numerous derivatives, such as those disclosed, for example, in EP 0 353 123, EP 0 356 271, EP 0 408442, EP 0 522 964, EP 0 420 707, EP 0 459 890 and EP 0 519 819, have already been proposed.

Clinical studies have demonstrated that PGF2$\alpha$ analogues have the property to let body hairs and eyelashes grow in man and animals (Murray A. and Johnstone M. D., 1997, Am. J. Opht., 124(4), 544-547). In man, tests carried out on the scalp have shown that a prostaglandin E2 analogue (viprostol) has the property of increasing hair density (Roenigk H. H., 1988, Clinic Dermatol., 6(4), 119-121). Furthermore, WO 98/33497 discloses pharmaceutical compositions comprising prostaglandins or prostaglandin derivatives against hair loss.

Prostaglandins and derivatives which are known to have a beneficial effect on hair growth are listed herein: prostaglandin A2, prostaglandin F2, prostaglandin E1, prostaglandin E2, Arbaprostil, Carboprost, Enprostil, Bimatoprost, Bemeprost, Latanaoprost, Limaprost, Misoprostol, Ornoprostil, Prostacyclin, Prostaglandin E1, Prostaglandin E2, Prostaglandin F2$\alpha$, Rioprostil, Rosaprostol, Sulprostone, Travaprost, Trimoprostil, Viprostol, 15-PGDH inhibitors.

An important observation documented with the use of prostaglandin analogues in the treatment of glaucoma was the stimulating effects of the drugs on eyebrow and eyelash hair growth and pigmentation; more specifically, observations of increased eyelash growth, thickness, luster and pigmentation; see, e.g., U.S. Pat. No. 6,262,105. In view of this interesting finding, these and other Prostaglandin analogues have been further evaluated for their ability to promote hair growth, including eyelash growth/enhancement; see, e.g., Wolf, et al., 2003 Dermatology Online Journal 9(3): 7, and references cited therein.

While prostaglandin or its derivatives seemingly excellent candidates to become a drug of choice in promoting eyelash growth, there are several important and serious considerations to address when evaluating the long term safety and overall practicality. For example, additional observations associated with the use of prostaglandin analogues to treat glaucoma were darkening of the iris (colored part) and periorbital tissue of the treated eye (the change noticeable usually within several months or years from the start of treatment with the prostaglandin analog), as well as increased pigmentation of eyelashes and eyelid skin darkening (US20080275118A1). Additionally, prostagladin and its analogues derived products can cause irritating, watery and/ or itchy eyes, headaches, allergies and/ or skin rash. Therefore, an alternative product is needed which does not have those undesired effects or side effects.

The German Federal Institute for Risk Assessment (Bundesinstitut für Risikobewertung in Deutschland) decided in its 13th session that cosmetics for eye lash growth comprising prostaglandin derivatives are not cosmetic products, but medication products according to §2 AMG of the German law. Therefore, the commercial sales and the advertisement of cosmetic eyelash growth products containing prostaglandin derivatives are forbidden according to German law.

Besides, nowadays consumers rather prefer natural cosmetic products, also for hair growth products, so that there is an increasing demand to find compositions without the use of synthetic active ingredients. Several natural

ingredients have been proposed for the use in promoting hair growth and treating hair loss (US 5 827 510 A; Fong et al., 2015 Journal of Ethnopharmacology, 175: 470; JP S59 152307 A; Semchin et al., 2016 Annals of Dermatology, 28(1): 55; JP 2006 273754 A).

The invention, therefore, proposes a composition based on natural active ingredients. However, natural active ingredients proven to promote hair growth, preferably eyelash growth, remain a scarcity.

## SUMMARY OF THE INVENTION

The objective of the invention is to enhance hair growth, eyebrow growth and/ or eyelash growth, without the use of prostaglandins and/ or derivatives thereof.

Another objective of the invention is to promote hair growth by only using natural active ingredients, preferably without the use of preservatives. It is an objective of the invention to minimize side effects or undesired effects compared to other hair growth products that are known from their active ingredients.

To achieve one or more objectives, several inventive compositions comprising ricinoleic acid and arachidonate, ricinoleic acid and diosgenin or ricinoleic acid, arachidonate and diosgenin are disclosed. The invention also relates to a composition that can optionally comprise a concentrated secretome of totipotent cells from Curcuma longa rhizome, which is commercially available under the tradename Capilia Longa produced by VYTRUS BIOTECH S. L. located in Spain.

The inventive composition does not trigger side effects such as irritating, watery and/ or itchy eyes, headaches, allergies and/ or skin rash, or other undesired effects from prostagladin analogue products.

The inventive composition can be a cream, serum or similar, which the patient or consumer can apply himself/herself to enhance hair growth at the site where the cream or serum was applied. Therefore, it is an objective of the invention to provide an easy and safe method to a patient or consumer to promote hair growth.

Furthermore, ricinoleic acid, arachidonate and diosgenin are natural active ingredients. Ricinoleic acid is obtained from castor oil, arachidonic acid is a polyunsaturated fatty acid present in the phospholipids (especially phosphatidylethanolamine, phosphatidylcholine, and phosphatidylinositides) of membranes of the animal cells, and diosgenin is obtained from the yam root.

Additionally, the inventive composition has the effect of giving smoothness, shine and care to hair when applied on it. Optionally the inventive composition can help against unpleasant effects of menopause.

The inventive composition comprises

a. 0.01 wt.%- 2.0 wt.%, preferably 0.05 wt.% - 0.5 wt.%, ricinoleic acid and/ or its salt based on the total weight of the composition, and
b. 0.01 wt.% - 2.0 wt.%, preferably 0.05 wt.% - 0.5 wt.%, arachidonate based on the total weight of the composition, and optionally
c. 0.01 wt.% - 2.0 wt.%, preferably 0.05 wt.% - 0.5 wt.% diosgenin based on the total weight of the composition.

In another aspect, the inventive composition comprises

a. 0.01 wt.%- 2.0 wt.%, preferably 0.05 wt.% - 0.5 wt.%, ricinoleic acid and/ or its salt based on the total weight of the composition, and
b. 0.01 wt.% - 2.0 wt.%, preferably 0.05 wt.% - 0.5 wt.% diosgenin based on the total weight of the composition, and optionally
c. 0.01 wt.% - 2.0 wt.%, preferably 0.05 wt.% - 0.5 wt.%, arachidonate based on the total weight of the composition.

In a particular aspect, the inventive composition is used as a cosmetic.

In another aspect, the inventive composition is used as a medicament.

## DEFINITIONS

### Ricinoleic acid

Ricinoleic acid, formally called 12-hydroxy-9-cis-octadecenoic acid is a fatty acid. It is an unsaturated omega-9 fatty acid and a hydroxyl acid. It is a major component of the seed oil obtained from mature Castor plant (Ricinus communis L., Euphorbiaceae) seeds or in sclerotium of ergot (Claviceps purpurea Tul., Clavicipitaceae). About 90% of the fatty acid content in castor oil is the triglyceride formed from ricinoleic acid. (Chemical Structure 1)

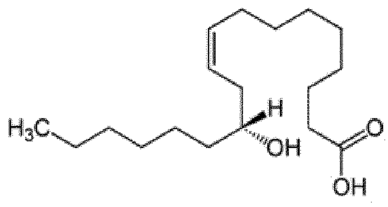

Chemical Structure 1: Ricinoleic acid

Ricinoleic acid is manufactured for industries by saponification or fractional distillation of hydrolysed castor oil. Ricinoleic acid may be present as a salt of the acid. As the salt, ricinoleic acid is present as ricinolate, where the acid has been deprotonated. In the case of the salt, a cation from the 1st or 2nd main group of the Periodic Table of the Elements, like sodium, potassium, lithium, magnesium or calcium is present.

**Arachidonic acid**

Arachidonic acid (AA, sometimes ARA) is a polyunsaturated omega-6 fatty acid 20:4($\omega$-6), or 20:4(5,8,11,14) (Chemical Structure 2).

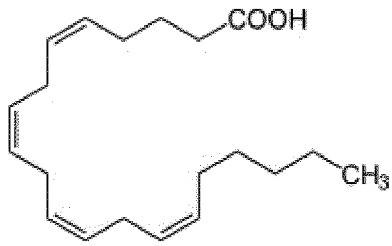

Chemical Structure 2: Arachidonic acid

Some chemistry sources define 'arachidonic acid' to designate any of the eicosatetraenoic acids. However, almost all writings in biology, medicine, and nutrition limit the term to all cis-5,8,11,14-eicosatetraenoic acid.

The arachidonic acid may be present as a salt of the acid. As a salt the arachidonic acid is present as arachidonate, which is an anion where the acid has been deprotonated. In the case of the salt, a cation from the 1st or 2nd main group of the Periodic Table of the Elements like sodium, potassium, lithium, magnesium and/ or calcium is present.

**Diosgenin**

Diosgenin (Chemical Structure 3), a phytosteroid sapogenin, is the product of hydrolysis by acids, strong bases, or enzymes of saponins, extracted from the tubers of Dioscorea wild yam, such as the Kokoro. The sugar-free (aglycone) product of such hydrolysis, diosgenin is used for the commercial synthesis of cortisone, pregnenolone, progesterone, and other steroid products.

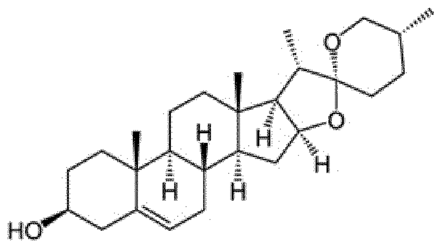

Chemical Structure 3: Diosgenin

**Secretome of totipotent cells from the rhizome of Curcuma longa (marketed as Capilia Longa)**

Curcuma longa is a tropical and subtropical plant characterized by the existence of very ramified, cylindrical and

orange rhizomes. These rhizomes are a modified root that acts as a storage and resistance organ. They grow endlessly and have excellent regenerative properties. Rich in curcuminoids (mainly curcumin), Curcuma longa is the most studied plant in biomedicine.

CAPILIA LONGA represents a new activity profile for this species describing the Phyto-Peptidic Fractions™ as a new range of bioactives from Curcuma longa (Curcumin is only the 2-5% of turmeric powder).

Capilia Longa is the concentrated secretome of totipotent cells from the rhizome of Curcuma longa. This secretome is rich in signalling peptides specially designed to create the optimal micro-environment to re-activate the hair growth. Further information about Capilia Longa and its active ingredients can be found in the WO2017/178250 A1 patent application. The patent application discloses that several plant extracts, preferably Curcuma Longa cell-free supernatant, more preferably a peptide solution comprising $CH_3$-C(O)-YIYT-$NH_2$ and/ or $CH_3$-C(O)-YIYTQ-$NH_2$ are beneficial for hair growth. However, an effect that the smoothness, shine or care of hair is improved by using Capilia Longa has not been observed.

## Cosmetics

Cosmetics are products used to enhance or change the appearance of the face, fragrance or the texture of the body. Many cosmetics are designed for use of applying to the face and body. They are generally mixtures of chemical compounds derived from natural sources (such as coconut oil), or may be synthetic or artificial.

In the United States, the Food and Drug Administration (FDA), which regulates cosmetics, defines cosmetics as "intended to be applied to the human body for cleansing, beautifying, promoting attractiveness, or altering the appearance without affecting the body's structure or functions". This broad definition includes any material intended for use as an ingredient of a cosmetic product. In the European Union, the manufacture, labelling, and supply of cosmetics and personal care products are regulated by Regulation EC 1223/2009.

Certain product ingredients are prohibited under Chapter IV of EC 1223/2009 based on their threat to human health. The Annexes are lists of chemical substances that are either banned, restricted or distinctively allowed for use in specific product types or to certain limits in the final product. These are as follows:

Annex II List of substances prohibited in cosmetic products
Annex III List of substances, which colour cosmetics should not contain except subject to the restrictions laid down
Annex IV List of colorants allowed in cosmetic products
Annex V List of preservatives allowed in cosmetic products
Annex VI List of UV filter allowed in cosmetic products

Regulation EC 1223/2009 applies to all the countries of the EU as well as Iceland, Norway, and Switzerland.

## Human Follicle Dermal Papilla Cells (HFDPC)

Human Follicle Dermal Papilla Cells (HFDPC) are primary cells isolated from human dermis of the scalp, originating from the occipital or temple region. HFDPC stain positive for alkaline phosphatase. Dermal papillae are embedded in a laminin and collagen IV rich extracellular matrix at the base of the hair follicles. HFDPC are used in vitro to predict their behaviour in vivo. The HFDPC used to present the effect of the inventive composition are based on primary cells, which are cultured directly from a subject. Therefore, it is highly likely that the in vivo behaviour of a composition, such as the inventive composition, will be similar, preferably the same, as in vitro.

## BRIEF DESCRIPTION OF DRAWINGS

**FIG 1: Investigation of cell membrane integrity:** The analysis of cell membrane integrity was necessary to demonstrate a potentially damaging effect of the substances on the cell membrane (cytotoxicity). If the cell membrane is damaged, the lactadehydrogenase (LDH) present inside the cell is released into the cell culture supernatant. The resulting NADH/H+ reduces the tetrazolium salt present in the reaction mixture to formazan. This becomes visible by a colour change, which can be detected at an absorption of 450 nm (y-axis: OD 450 nm) in the microplate reader. The higher the intensity of the colour change, the greater the degree of membrane damage. A low OD 450 nm value is desired.

Four diagrams are presented: The LDH level after one week (LDH $1^{st}$ Week of Trial), two weeks (LDH $2^{nd}$ Week of Trial) and three weeks (LDH $3^{rd}$ Week of Trial) after the HFDPC cells were exposed to the control conditions or to

the actives substances.

The control conditions are HFDPC with medium (blue, C+M). Active substances are arachidonate with low concentration (purple, Ara 1.c), arachidonate with high concentration (orange, Ara 2.c), diosgenin with low concentration (black, Dios 1.c), diosgenin with high concentration (brown, Ara 2.c), ricinoleic acid with low concentration (dark blue, Rici 1.c), ricinoleic acid with high concentration (dark violet, Rici 2.c), inventive example with low concentration (red, IE 1.c) inventive example with high concentration (green, IE 2.c).

The fourth diagram presents a background control, in which the LDH level was measured by OD 450 nm values (y-axis) from the cell culture supernatant for the medium (blue, M) as a control test and for the medium with the active substance in high concentrations (green, M+Ara 2.c; purple, M+Dios 2.c; orange, M+Rici 2.c; black, M+IE 2.c) after one, two and three weeks (x-axis).

**FIG 2: Phase contrast images of untreated HFDPC (HFDPC in medium) 1 week after cell cultivation**

a) 10x magnification, b) 20x magnification

**FIG 3: Phase contrast images of treated HFDPC in 20x magnification, one week after addition of the active ingredient**

a) Ara 1.c, b) Ara 2.c, c) Rici 1.c, d) Rici 2.c, e) Dios 1.c, f) Dios 2.c, g) IE 1.c, h) IE 2.c

**FIG 4: Phase contrast images of untreated and treated HFDPC in 20x magnification, two weeks after addition of the active ingredient**

a) Medium b) Ara 1.c, c) Ara 2.c, d) Rici 1.c, e) Rici 3.c, f) Dios 1.c, g) Dios 2.c, h) IE 1.c, i) IE 2.c

**FIG 5: Phase contrast images of untreated and treated HFDPC in 20x magnification, three weeks after addition of the active ingredient**

a) Medium b) Ara 1.c, c) Ara 2.c, d) Rici 1.c, e) Rici 3.c, f) Dios 1.c, g) Dios 2.c, h) IE 1.c, i) IE 2.c

**FIG 6: Alkaline Phosphatase**

Alkaline phosphatase (AP) is an enzyme that catalyses the hydrolysis of a large number of phosphate esters. The enzyme is formed by the HFDPCs during the hair cycle. Thus, the detection of AP is the proof of hair growth. The alkaline phosphatase expressed by the cells was stained with the alkaline phosphatase staining kit from abcam. A higher value for the coloured spots given in percent (y-axis) means a higher value of alkaline phosphatase. A higher alkaline phosphatase value is an indication for more hair growth.

Three diagrams are presented: The alkaline phosphatase level (y-axis) of HFDPC after one week (Alkaline Phosphatase 1$^{st}$ Week of Trial), after two weeks (Alkaline Phosphatase 2$^{nd}$ Week of Trial) and after three weeks (Alkaline Phosphatase 3$^{rd}$ Week of Trial) when exposed to the active substances.

The control conditions are HFDPC with medium (blue, C+M). Active substances are arachidonate with low concentration (purple, Ara 1.c), arachidonate with high concentration (orange, Ara 2.c), diosgenin with low concentration (black, Dios 1.c), diosgenin with high concentration (brown, Ara 2.c), ricinoleic acid with low concentration (dark blue, Rici 1.c), ricinoleic acid with high concentration (dark violet, Rici 2.c), inventive example with low concentration (red, IE 1.c) inventive example with high concentration (green, IE 2.c).

**FIG 7: Results of the cell count after trypsination**

To perform this investigation 20000 cells per well were seeded in the 24-wells with the addition of a maximum of 1 mL medium. The cells were seeded for seven days and then the cells were exposed to each active ingredient or composition. After 7 days (one week), 14 days (two weeks) and 21 days (three weeks) the cells were trypsinized and counted. The cells for control tests were grown for one week, two weeks and three weeks in pure cell culture medium. Four diagrams are presented: Each diagram shows the number of cells per well (y-axis: Cells / Well) after one week, two weeks or three weeks (x-axis) for a specific active substance. The first diagram shows the values for arachidonate with low concentration (purple, Ara 1.c), arachidonate with high concentration (orange, Ara 2.c) and the control test (blue, C+M). The second diagram shows the values for diosgenin with low concentration (black, Dios 1.c), diosgenin with high concentration (brown, Ara 2.c) and the control test (blue, C+M). The third diagram shows the values for ricinoleic acid with low concentration (dark blue, Rici 1.c), ricinoleic acid with high concentration (dark violet, Rici 2.c) and the control test (blue, C+M).

The fourth diagram shows the values for inventive example with low concentration (red, IE 1.c) inventive example

with high concentration (green, IE 2.c) and the control test (blue, C+M).

**FIG 8: Characterization of HFDPC by expression of the marker vimentin by immunocytochemistry staining, one week after addition of the active ingredients**

Cells were fixed with formaldehyde and labeled with anti vimentin/anti rabbit AlexaFluor 555 (red).
a) medium, b) medium without primary antibodies, c) Ara 1.c, d) Ara 2.c, e) Rici 1.c, f) Rici 2.c, g) Dios 1.c, h) Dios 2.c, i) IE 1.c, j) IE 2.c.

**FIG 9: Characterization of HFDPC by expression of the marker vimentin by immunocytochemistry and DAPI staining, three weeks after addition of the active ingredients**

Cells were fixed with formaldehyde and labeled with anti vimentin/anti rabbit AlexaFluor 555 (red). The cell core staining was done with DAPI (blue).
a) medium, b) medium without primary antibodies, c) Ara 1.c, d) Ara 2.c, e) Rici 1.c, f) Rici 2.c, g) Dios 1.c, h) Dios 2.c, i) IE 1.c, j) IE 2.c

**FIG 10: VEGF-ELISA (VEGF Secretion)**

VEGF belongs to the signal molecules and stimulates the proliferation and migration of HFDPC. HFDPC generate more VEGF in the anagen phase. Thus, VEGF is an indication of the anagen phase of a hair. The detection of the VEGF protein was performed with the Human VEGF ELISA Kit from Invitrogen. The procedure was performed according to the protocol contained in the test kit with the cell supernatants previously frozen at -80°C. The removal of the cell culture supernatants was carried out on a 24-well scale. A higher value for the VEGF [pg/ml] (y-axis) means a higher value of detected VEGF. A higher VEGF value is an indication for a longer anagen phase. A longer anagen phase is an indication for more hair growth.

Three diagrams are presented: The VEGF level (y-axis) of HFDPC after one week (VEGF 1$^{st}$ Week of Trial), after two weeks (VEGF 2$^{nd}$ Week of Trial) and after three weeks (VEGF 3$^{rd}$ Week of Trial) when exposed to the active substances.

The control conditions are HFDPC with medium (blue, C+M). Active substances are arachidonate with low concentration (purple, Ara 1.c), arachidonate with high concentration (orange, Ara 2.c), diosgenin with low concentration (black, Dios 1.c), diosgenin with high concentration (brown, Ara 2.c), ricinoleic acid with low concentration (dark blue, Rici 1.c), ricinoleic acid with high concentration (dark violet, Rici 2.c), inventive example with low concentration (red, IE 1.c) inventive example with high concentration (green, IE 2.c).

**FIG 11: Cell impedance measurements**

The influence of various substances on the growth of HDPFC was investigated with the aid of a cell impedance device (xCELLigence® RTCA DP).
To perform this study 5000 HFDPC (Passage: 5) per well were seeded in the 16-well wells, coated on 50 µL collagen with the addition of a maximum of 200 µl medium. The cells were grown for 96 h in pure cell culture medium. After 96 h the "pure" cell culture medium was completely removed and medium with the respective additives was added. A further change with the respective additives took place after 48 h.
The CI (y-axis) is thus a measure of cell proliferation, cell morphology and cell vitality (only vital, adherent HFDPC can proliferate) over time (x-axis).
The presented diagram shows Cell Index (CI) values for control conditions with only HFDPC in medium (pink, Control test). Otherwise tested active substances are arachidonate with 50 µg/ml concentration (blue), diosgenin with 50 µg/ml concentration (black), diosgenin with 500 µg/ml concentration (green), ricinoleic acid with 100 µg/ml concentration (sky blue), diosgenin with 100 µg/ml concentration (brown), diosgenin with 250 µg/ml concentration (purple), ricinoleic acid with 100 µg/ml concentration (light blue), ricinoleic acid with 500 µg/ml concentration (orange), arachidonate with 100 µg/ml concentration (green), arachidonate with 250 µg/ml concentration (red).

**FIG 12: Increase of eyelash growth tested on a human**
The eyelash length was at 4 mm of a female human test person at the beginning of the evaluation. During the 5 weeks evaluation of the product composition on a female test person, the left eyelash (A) was not treated with the product

composition and the right eyelash was treated with the product composition by applying it on the eyelash. The eyelash length of the left eyelash remained at 4 mm, whereas the right eyelash increased to 8 mm. Therefore, after 5 weeks the right eyelash length could be increased by 100% with the use of the product composition and the eyelashes became also thicker.

## DETAILED DESCRIPTION OF THE INVENTION

In one aspect, the inventive composition comprises

a. in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt. %, ricinoleic acid and/ or its salt based on the total weight of the composition, and
b. in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt.%, arachidonate based on the total weight of the composition.

The inventive composition comprising ricinoleic acid and/ or its salt and arachidonate was surprisingly found to have a beneficial effect on hair growth of humans. The effect of hair growth is higher when ricinoleic acid and/ or its salt and arachidonate are combined than using the ricinoleic acid and/ or its salt or arachidonate alone.

The effect of hair growth can be measured and analyzed on humans or on HFDPC. Suitable parameters for HFDPC to investigate hair growth are the examination of cell proliferation (cell index CI), the assessment of cell morphology (marker of cytotoxicity), the staining of alkaline phosphatase (marker: cell function/ hair growth), the immunofluorescent staining of the HFDPC (cell verification).

In a preferred embodiment the cell functionality, preferably HFDPC, was measured by VEGF and/ or alkaline phosphatase testing. The inventive composition preferably does not influence the VEGF secretion of HFDPC compared to a control test in which HFDPC is treated without the active substance. In another embodiment, the inventive composition preferably has a positive influence on alkaline phosphatase secretion of HFDPC.

In preferred embodiment, the inventive composition is cell-compatible. Cell-compatibility can be tested by means of cell morphology, cell index, LDH and/ or cell staining with vimentin. Therefore, the cell-compatible inventive composition is given to cells, preferably HFDPC, and then tested by cell morphology, cell index, LDH and/ or cell staining with vimentin. The inventive composition preferably is in all those cell-compatibility test similar or better compared to a control test under the same conditions but without the inventive composition.

For the inventive composition comprising ricinoleic acid and/ or its salt and arachidonate, the ratio of ricinoleic acid versus arachidonate in the composition is generally from 1:2 to 2:1, preferably from 1:1.5 to 1.5:1. A composition within those indicated ratios of ricinoleic acid versus arachidonate was found to be beneficial for hair growth or HFDPC proliferation. A composition of ricinoleic acid and diosgenin not underlying the stated ratio was found to not stimulate hair growth HFDPC proliferation.

In a different aspect, the inventive composition comprises

a. in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt.%, ricinoleic acid and/ or its salt based on the total weight of the composition, and
b. in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt.%, diosgenin based on the total weight of the composition.

The inventive composition comprising ricinoleic acid and/ or its salt and diosgenin was found to have a beneficial effect on hair growth of humans. The effect of hair growth is higher when ricinoleic acid and/ or its salt and arachidonate are combined than using the ricinoleic acid and/ or its salt or diosgenin alone. The effect of hair growth can be measured and analyzed on humans or HFDPC.

For the inventive composition comprising ricinoleic acid and/ or its salt and diosgenin, the ratio of ricinoleic acid versus diosgenin in the composition is generally from 1:2 to 2:1, preferably from 1:1.5 to 1.5:1. A composition of ricinoleic acid and diosgenin not underlying the stated ratio was found to not stimulate hair growth HFDPC proliferation.

In a specific aspect, the inventive composition comprises

a. in the range of 0.01 wt.% - 2.0 wt.%, preferably 0.025 wt.% - 1.5 wt.%, more preferably 0.05 wt.% - 0.5 wt.% most preferably 0.075 wt.% - 1.25 wt.%, ricinoleic acid and/ or its salt based on the total weight of the composition, and
b. in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt.%, arachidonate based on the total weight of the composition, and
c. in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt.%, diosgenin based on the total weight of the composition.

The preferred inventive composition comprising ricinoleic acid and/ or its salt, arachidonate and diosgenin was found to have a beneficial effect on the hair growth of humans. The effect of hair growth is higher when ricinoleic acid and/ or its salt, arachidonate and diosgenin are combined than using the ricinoleic acid and/ or its salt, arachidonate or diosgenin alone. The effect of hair growth can be measured and analyzed on humans or HFDPC.

For the inventive composition comprising ricinoleic acid and/ or its salt, arachidonate and diosgenin, the ratio from 4:1 to 1:1, preferably from 2.5:1 to 1.5:1, most preferably 2:1, of the combined amount of arachidonate and ricinoleic acid versus diosgenin in the composition. A composition of ricinoleic acid and/ or its salt, arachidonate and diosgenin not underlying the stated ratio was found to not stimulate hair growth, HFDPC proliferation.

The following features relate to every aspect of the composition of the present invention as described above or below.

The inventive composition preferably does not harm a Human Follicle Dermal Papilla Cell (HFDPC) for three weeks detected with a LDH cytotoxicity test.

The inventive composition preferably does not harm a Human Follicle Dermal Papilla Cell (HFDPC) for three weeks detected with a VEGF test.

The inventive composition preferably does not harm a Human Follicle Dermal Papilla Cell (HFDPC) for three weeks detected with an Alkaline Phosphatase test.

The compositions according to the invention can comprise a concentrated secretome of totipotent cells from *Curcuma longa* rhizome. In a preferable embodiment the inventive composition comprises in the range of 0.01 wt.% - 2.0 wt.%, preferably 0.025 wt.% - 1.5 wt.%, more preferably 0.05 wt.% - 0.5 wt.% most preferably 0.075 wt.% - 0.25 wt.%, concentrated secretome of totipotent cells from *Curcuma longa* rhizome based on the total weight of the composition.

The concentrated secretome of totipotent cells from *Curcuma longa* rhizome is commercially available under the name Capilia Longa. Further information about Capilia Longa and its active ingredients can be found in the WO2017/178250 A1 patent application. The addition of the concentrated secretome of totipotent cells from *Curcuma longa* rhizome has the effect, that the cell proliferation and hair growth can be further stimulated.

The inventive composition can comprise the peptides $CH_3$-C(O)- YIYT-$NH_2$, $CH_3$-C(O)-YIYTQ-$NH_2$ and/ or mixtures thereof. These peptides can be made synthetically or extracted from other plants, and can be added instead of or in addition to the concentrated secretome of totipotent cells from *Curcuma longa* rhizome. This will have the similar effect as using the concentrated secretome of totipotent cells from *Curcuma longa* rhizome, as can be gathered from WO2017/ 178250 A1.

The inventive composition preferably does not comprise any hormones. Hormones can cause side effects when applied on humans. Therefore, not using hormones in the inventive composition can minimize side effect, which are usually unwanted. On the other hand, the inventive composition can provide the necessary components, so that the human body forms hormones by itself.

The inventive composition can comprise vitamins. The addition of vitamins can be used to provide further functionalities to a human other than hair growth. In a preferred embodiment the composition comprises vitamin C, vitamin B1, vitamin B3, vitamin B5, vitamin B6, vitamin B2, vitamin B8, vitamin B9, derivatives thereof, and/ or mixtures thereof, more preferably vitamin C, vitamin E and vitamin B5 and derivatives thereof, most preferably vitamin C, vitamin E and vitamin B5.

Vitamins, preferably vitamin E or vitamin C, can be used as antioxidants in the inventive composition, so that the inventive composition can be stored for longer.

The inventive composition can further comprise additives, such as antioxidants, emulsifiers, preservatives, colorants, UV filters, acids, bases, pH stabilizers and perfumes. A list of additives can be found in Regulation EC 1223/2009 and its annexes. This list of banned or distinctively allowed additives should not limit the potential additives for the inventive composition.

In another preferred embodiment, the inventive composition can comprise isosorbide. Isosorbide has the effect that it induces lasting skin and/ or hair hydration, imparts a pleasant non tacky feel on the skin and acts as a strong humectant and moisturizing agent. Furthermore, isorbide can reduces growth of bacteria, yeast and fungi. However, isorbide is not listed on Chapter IV of EC 1223/2009 Annex V (List of preservatives allowed in cosmetic products). Isorbide is part of a plant-based microbiota-balancing active product, which can be used as a preservative, that is marketed as PO 500 by Beauté By Roquette.

In another preferred embodiment, the inventive composition can comprise preservatives. Preservatives have the effect, that the inventive composition can be used for several applications without high degradation of the inventive composition. Therefore, the use of preservatives in the inventive composition can simply and unburden the use of the inventive composition. In a more preferred embodiment, the inventive composition satisfies the criteria A and B for preservation efficacy test according to DIN EN ISO 11930.

The inventive composition can further comprise core ingredients, such as lipophilic substances, such as oil, wax and/ or fat, and/ or hydrophilic substances, such as water and/ or polar carbohydrates. In a preferred embodiment, the inventive composition comprises core ingredients and ricinoleic acid and/ or its salt, arachidonate and/ or diosgenin. The

core ingredients make it easier to distribute ricinoleic acid and/ or its salt evenly, arachidonate and/ or diosgenin on the surface of humans. The core ingredients have the beneficial effect that less ricinoleic acid and/ or its salt, arachidonate and/ or diosgenin need to be used without a noticeable decrease in hair growth. Furthermore, the core ingredients can moisturize and/ or grease the surface, preferably the skin, where it is applied on.

In another particular embodiment, the inventive composition can be an oil-in-water (O/W) emulsion or a water-in-oil (W/O) emulsion. Preferably, the inventive composition is an oil-in-water emulsion. An emulsion comprises any type of lipophilic substance, such as oil, wax and/ or fat, and any type of hydrophilic substance, such as water and/ or polar carbohydrates. Preferably, the emulsion of the inventive composition comprises mostly, like in a range of 40 to 70 wt.%, preferably in a range of 45 to 65 wt.%, more preferably in a range of 50 to 60 wt.% based on the overall weight of the composition, a hydrophilic substance, preferably water. An emulsion preferably comprises an emulator and optionally a stabilizer. In a preferred embodiment, the inventive composition is an emulsion comprising an emulator and a stabilizer. In a more preferred embodiment, the inventive composition is an oil-in-water emulsion comprising an emulator and a stabilizer. In a preferred embodiment the oil phase comprises almond oil and/ or castor oil. In more preferred embodiment the oil phase comprises in a range of 5 to 20 wt.%, preferably in a range of 7.5 to 15 wt.% more preferably in a range of 10 to 13 wt.% based on the overall weight of the composition almond oil. In another preferred embodiment the inventive composition comprises in a range of 15 to 40 wt.%, preferably in a range of 20 to 30 wt.% more preferably in a range of 22 to 27 wt.% based on the overall weight of the composition castor oil.

In a preferred embodiment, the ratio of hydrophilic substance to oil, preferably of water to castor oil, is between 3:1 to 1:1, preferably 2.5:1 to 2:1 based on the overall wt.% in the inventive composition. If the ratio does not follow the ratio a stable emulsion cannot be formed, which lasts longer than 1 day.

In another embodiment, the inventive composition does not comprise prostaglandin A2, prostaglandin F2, prostaglandin E1, prostaglandin E2, Arbaprostil, Carboprost, Enprostil, Bimatoprost, Bemeprost, Latanaoprost, Limaprost, Misoprostol, Ornoprostil, Prostacyclin, Prostaglandin E1, Prostaglandin E2, Prostaglandin F2$\alpha$, Rioprostil, Rosaprostol, Sulprostone, Travaprost, Trimoprostil, Viprostol and/ or 15-PGDH inhibitors. In a more preferred embodiment, the inventive composition does not comprise Latanoprost or Bismatoprost. Therefore, the inventive composition does not comprise any additional prostaglandin derivative to induce hair growth.

In another embodiment, the inventive composition does not comprise minoxidil and/ or finasterid. Minoxidil and finasterid are typical active ingredients to induce hair growth in commercially available hair growth products. The inventive composition does not comprise Minoxidil and/ or finasterid to induce hair growth.

In one aspect, the composition as described above or below is used as a cosmetic.

In a particular embodiment, the inventive composition is a cosmetic composition used as a cosmetic product for humans. Therefore, the inventive composition needs to comply with Regulation EC 1223/2009 so that it can be marketed in the European Union. In other countries, other regulations may apply, so that the inventive composition can be marketed as a cosmetic composition.

In a more preferred embodiment, the inventive composition is a cosmetic composition used by humans as a cosmetic product, for daily, weekly or any type of periodic usage.

The inventive composition is preferably used on the surface, preferably on the skin, of humans. In other words, the inventive composition is suitably a cosmetic product for the topical use on the skin of humans. The invention, therefore, relates to the cosmetic use of the inventive composition, wherein the inventive composition is applied on the skin of humans. The inventive composition is preferably used on the eyelid, eyebrow, chin and/ or scalp of humans. In other word, the composition relates to the use of the inventive composition, wherein the composition is applied on the eyelid, eyebrow, chin and/ or scalp of humans. In a preferred embodiment, the inventive composition is used on the eyelid and/ or eyebrow of humans. In a more preferred embodiment, the inventive composition is a cosmetic product used on the eyelid of humans. In a preferred embodiment, the inventive composition is applied on the eyelid with an tubular, nozzle or rod-shaped tool, like an applicator, wand, or brush. In another preferred embodiment the inventive composition can also have other cosmetic functionalities, such as darkening the eye lash or tidying the eye lash, as it is known for mascara or other beauty products.

The inventive composition is preferably used to stimulate or induce hair growth, preferably eyelash growth, eyebrow growth, beard growth and/ or scalp hair growth, more preferably eyelash growth and/ eyebrow growth, most preferably eyelash growth. In a more particular embodiment, the inventive composition is applied on the eyelid of humans to induce eyelash growth. Also, the invention relates to the cosmetic use of the inventive composition, wherein the composition stimulates hair growth, preferably eyelash growth, eyebrow growth, beard growth and/ or scalp hair growth. In a more preferred embodiment, the inventive composition, increases eyelash length when used on an eyelash, preferably by at least 50%, more preferably by at least 100%, like 50 - 150%, more preferably 100 - 130%, and/ or eyelash thickness preferably by at least 30%, more preferably by 50%.

In another aspect of the invention, the inventive composition is used as a medicament, preferably for humans. Thus, the inventive composition is not a cosmetic composition.

In a preferred embodiment of the invention, the medicament is used in a method to treat hair loss, preferably

hypotrichosis and/ or alopecia.

In a more preferred embodiment, the composition of the invention is used as a medicament used in a method to treat hair loss, preferably hypotrichosis and/ or alopecia of eyelashes, eyebrows, and scalp hair, preferably eyelashes and/ eyebrows, more preferably eyelashes of humans.

With medicinal treatment, it is meant that hair growth can be regenerated, hair loss can be stopped and/ or hair loss can be delayed.

In a preferred embodiment, the inventive composition when used as a medicament in a method for the treatment of hypotrichosis, is applied on the skin of a human. The topical medication of the inventive composition has the advantage that hair growth will only be induced on the distinct areas, like eyelashes, eye brows, chin and/ or scalp, preferably eyelids and/ eye brows, most preferably eyelids, where the inventive composition was applied.

In another embodiment, the inventive composition is applied on the skin of a human and is remained on the skin of a human for more than 30 min, preferably more than 3 h, but not more than 8 h, like in a range from 30 min to 8 h, preferably in a range from 3 h to 8 h.

The time is counted as soon as the inventive composition is applied and distributed on the surface, where the hair growth on the human is desired. The time count stops, when the inventive composition is removed, like through washing away or wiping away. In the time period where the inventive composition remains on the skin of the human, the inventive composition can undergo changes. The components of the inventive composition can be absorbed by the human skin, evaporate to the atmosphere, or degrade.

The invention is described in more detail by means of the following examples.

## EXAMPLES

### General procedure for cells:

First, Human Follicle Dermal Papilla Cells (HFDPC) cells were thawed and seeded into collagen-coated cell culture flasks (T25). Then the medium was changed every 48 h. After 4 days the cells were trypsinized and seeded in T75 cell culture flask. In preparation for the final experiment, the cells were trypsinized and then counted with a cell counter. The cell concentration was then adjusted in a defined amount of medium followed by seeding of the cells in 96-well plates for the subsequent cell impedance measurements (5000 cells per well) or in 24-well plates (20000 cells per well). Subsequently, the cells were cultivated for 7 days in pure culture medium with regular medium changes after 2-3 days. After 7 days, the medium with the additives listed in Table 1 was added (designated as day 0), followed by a 3-week cultivation of the cells. The medium (and addition of fresh active substances) was changed every 2-3 days. The timing of the medium exchange was strictly adhered to ensure the accumulation of possible substances in the medium and to make the data comparable.

The following examinations were carried out after 7, 14 and 21 days.

Cells in pure culture medium (C+M) were used as controls (untreated).

All cell culture substrates were coated with collagen prior to cell seeding.

Table 1:

| Component | 1. Concentration | 2. Concentration |
|---|---|---|
| Sodium Arachidonate (Ara) | 1 μg/mL (Ara 1c) | 2 μg/mL (Ara 2c) |
| Ricinolic acid (Rici) | 1 μg/mL (Rici 1c) | 5 μg/mL (Rici 2c) |
| Diosgenin (Dios) (conc. >99.9% extracted from Yams) | 5 μg/mL (Dios 1c) | 20 μg/mL (Dios 2c) |
| Inventive Example Concentration of each component (Arachidonate, Diosgenin, Ricinolic acid) | IE 1c | IE 2c |

### Results:

### 1. Examination of cell membrane integrity (marker of cytotoxicity):

The analysis of cell membrane integrity was necessary to demonstrate a potentially damaging effect of the substances on the cell membrane (cytotoxicity). If the cell membrane is damaged, the lactadehydrogenase (LDH) present inside the cell is released into the cell culture supernatant. The enzyme is detected by using the LDH-Cytotoxicity Colorimetric Assay Kit II (Biovision). As an enzyme in the supernatant, LDH catalyses the conversion of pyruvate to lactate with simultaneous conversion of NADH/H+ and NAD+. The resulting NADH/H+ reduces the tetrazolium salt present in the reaction mixture to formazan. This becomes visible by a colour change, which can be detected at an absorption of 450 nm

in the microplate reader. The higher the intensity of the colour change, the greater the degree of membrane damage. The investigation of the cell membrane integrity was performed on a 24-well scale.

The detection of LDH in the cell culture supernatant is a measure of cell membrane damage. The higher the degree of damage, the more LDH can be detected in the cell culture supernatant (visible by the increase in optical density/OD). Cells cultivated in pure medium were used as controls. Already after 7 days it was shown that the addition of the active substances to the cells did not induce any significant damage to the cell membrane (in direct comparison to untreated cells) (FIG 1, LDH 1$^{st}$ Week of Trial). Furthermore, cells treated with the two inventive examples (IE 1.c and IE 2.c) showed a significantly reduced release of the enzyme into the cell culture supernatant. This indicates that the cultivation of the cells after the addition of the active substances leads to significantly improved cultivation conditions. After 2 weeks, this significant effect was visible for all active ingredients and thus clearly showed that the cultivation of the cells is significantly positively influenced after addition of the active ingredients (FIG 1, LDH 2$^{nd}$ Week of Trial).

After 3 weeks no difference between untreated and drug-treated HFDPC (and thus no cytotoxic effect) was found (FIG 1, LDH 2$^{nd}$ Week of Trial).

Therefore, it can be concluded that the addition of the inventive examples (IE 1.c and IE 2.c) do not induce any damage to the cell membrane. On the contrary, an improved cultivation of the cells was shown. Thus, the inventive examples are not cytotoxic.

The following diagrams show the course of LDH release. As background control the LDH release in the pure cell culture supernatant is shown. Despite the addition of the active substance to the medium, the background values are all comparable and were not subtracted separately from the detected values. (FIG 1)

**2. Assessment of cell morphology (marker of cytotoxicity):**

Cell morphology was assessed through microscopic examination of the cells with an inverted microscope (phase contrast microscopy) at 10x and 20x primary magnification. The morphology of the cells provides information on the adherence behaviour of the cells,possible cell activation and cell stress. This examination was carried out on a 24-well scale.

Cells after 1 week of cultivation (with and without active ingredients)

Adherent HFDPC in pure cell culture medium (control cells) showed a morphologically heterogeneous appearance (FIG 2). They had a 35 - 200 μm longish to round shape and usually had several pointed cell outlets, which spread in different directions. In the cytoplasm neither nucleus nor vacuoles were visible in the phase contrast. The differentiation of the cell bodies from their surroundings was very clear.

**2.a Cells after addition of the active substance (after 1 week):**

One week after the addition of the active ingredients, the cells treated with arachidonate showed a similar cell shape to the untreated cells. A greater confluence was seen in the treatment with diosgenin, ricinoleic acid and the combination active ingredient. In addition, it was observed that HFDPC tended to grow elongated at a higher confluence. Within one group of active ingredients there were hardly or only slight differences. (FIG 3)

**2.b Cells after addition of the active substance (after 2 weeks):**

In the second week of the experiment, untreated cells showed a round cell shape and fine, fibrous cell extensions. The cells treated with arachidonic acid showed almost the same morphology. The higher concentration of arachidonic acid also resulted in a higher cell density. The addition of diosgenin, ricinoleic acid and the combination of all three active ingredients resulted in a very high confluence in the well and elongated, typical growth, although this was somewhat more pronounced in ricinoleic acid and the combination approaches compared to the addition of diosgenin. (FIG 4)

**2.c Cells after addition of the active substance (after 3 weeks):**

Untreated cells had a roundish shape with a non-uniform border. There was no confluent monolayer in the respective wells. A similar picture was found with arachidonic acid-treated cells in both concentrations. Cells treated with ricinoleic acid (both concentrations) and the combination approaches showed a confluent, very dense monolayer and an elongated cell shape. This picture was also seen in cells treated with diosgenin in the higher combination. A treatment with diosgenin in the lower concentration led to an appearance, which resembled untreated cells - round, not evenly delimited cell bodies. (FIG 5)

In summary, the addition of ricinoleic acid, diosgenin (concentration-dependent) and the combination of the three agents were very beneficial for cell adherence and proliferation.

This became visible by the confluence of the colonized 24-well cavities after 3 weeks and the cell shape. As these

cells are very sensitive cells (see untreated cells), we can speak of a great success with this parameter alone. It was clearly shown that the addition of the active ingredients led to a significant improvement of the culture conditions with an increased cell proliferation compared to pure cell culture medium.

### 3. Staining of alkaline phosphatase (marker: cell function/ hair growth)

Alkaline phosphatase (AP) is an enzyme that catalyses the hydrolysis of a large number of phosphate esters. The enzyme is formed by the HFDPCs during the hair cycle. Thus, the detection of AP is the proof of hair growth. The alkaline phosphatase expressed by the cells was stained with the alkaline phosphatase staining kit from abcam. The staining was performed according to the enclosed protocol from abcam. The result was recorded with an inverted light microscope. The evaluation was performed with ImageJ by determining the percentage of the stained area to the total area of the image.
This analysis was performed to investigate cell function in order to draw possible conclusions about the induction of hair formation in vivo. It could be shown that a significantly higher expression of the alkaline phosphatase occurred for all treated HFDPC versus untreated HFDPC. In the $1^{st}$, $2^{nd}$ and $3^{rd}$ week of trial it can be seen that the inventive examples (IE 1.c and 2.c) performed the best. IE 1.c presented 3x higher expression than the untreated HFDPC or Ara 1.c treated cell cultures. IE 1.c treated HFDPC expressed 100% more than Dios 1.c and around 70% more Rici 1.c.
After 3 weeks of trial the inventive examples (IE 1.c and 2.c) performed the best in the alkaline phosphatase test compared to other treated and untreated HFDPC. IE 1.c
In summary, it can be stated that all additives had a positive influence on the expression of alkaline phosphatase. Since alkaline phosphatase is produced by HFDPC cells during the hair cycle, the detection of alkaline phosphatase in vitro is a significant indication that hair growth could be induced in vivo. (FIG 6)

### 4. VEGF-ELISA (Marker: cell function/hair growth):

VEGF belongs to the signal molecules and stimulates the proliferation and migration of HFDPC. HFDPC generate more VEGF in the anagen phase. Thus VEGF is an indication of the anagen phase of a hair. From this it can be concluded that hair formation is induced and/ or prolonged. The detection of the VEGF protein was performed with the Human VEGF ELISA Kit from invitrogen. The procedure was performed according to the protocol contained in the test kit with the cell supernatants previously frozen at -80°C. The removal of the cell culture supernatants was carried out on a 24-well scale.
VEGF was analyzed in the cell culture supernatant to check whether the active ingredients have an influence on cell function and, if so, whether they promote the release of VEGF (see figure). In vivo, this would contribute to improved hair growth, especially of the eyelashes.
Throughout the entire duration of the experiment it was shown that there was no significant increase in VEGF secretion after addition of the various active ingredients by HFDPC (FIG 10).
On the other hand, it was shown that a lower VEGF release of HFDPC could be detected for the following:

1. dios 1c- after 1 week
2. dios 1c - after 3 weeks
3. ric 2.c - after 3 weeks
4. Ara 1c - after 3 weeks

This low VEGF release occurred with the addition of Dios and Ara depending on concentration and time, whereby the influence on VEGF release occurred in each case at the lower concentration of the added active substance.
It should be noted that the inventive compositions had no negative influence on VEGF secretion compared to the control sample.
Therefore, it can be concluded that this influence was mainly due to cell aging, as can be seen from the experiments with alkaline phosphatase.
In summary, it can be concluded that the addition of the individual active ingredients (alone or in combination) had a minor influence on cell function in some cases, but no drastic cell changes were observed in VEGF secretion. Thus, the treated cells are exposed to the inventive composition for the same length of time in the anagen phase (see VEGF results), but the cell function is much longer (see alkaline phosphatase), which leads to the conclusion that hair growth increases.

### 5. Measurement of cell growth (marker of cytotoxicity) by means of cell counting:

This investigation was performed on a 24-well scale.
To perform this investigation 5000 cells per well were seeded in the 96-wells with the addition of a maximum of 200 µl medium. The cells were grown for 7 days in pure cell culture medium. The medium was changed every 2-3 days,

whereby 100 µl of old medium was removed and replaced by 100 µl of new medium. After 7 days the "pure" cell culture medium was completely removed and medium with the respective additives was added. The cells were then cultivated for 3 weeks with regular replacement of the medium with the respective additives.

Cell counting after trypsinizing: The exact cell count of HFDPC was determined after trypsination of the cells in the respective well and uptake of the resulting cell pellets in medium. All pellets of one sample were combined in a cell culture vessel and then counted with a cell counter.

It was shown that the addition of arachidonate (independent of time and concentration) did not negatively affect the cell count over the entire duration of the experiment. The cell count remained constant over 2 weeks. After 3 weeks there was an increase in HFDPC after addition of Ara in the higher concentration compared to untreated cells.

The addition of diosgenin led to a positive influence on the cell number in comparison to untreated cells, depending on concentration and time. Diosgenin 2c led to a significant increase in cell number over time compared to untreated and Diosgenin 1c treated cells. The number of HDFPC remained constant after addition of Diosgenin 1c, but was increased compared to untreated cells.

The addition of ricinoleic acid led to a significant increase in cell count depending on time and concentration. Already after one week the difference was visible in both treated groups compared to untreated cells. This increase in cell number increased dramatically over time and was significantly higher compared to untreated cells.

The combined addition of all active ingredients did not lead to a significant change in cell count after 1 week when IE 2c was added. The addition of IE 1c led to a significant increase compared to untreated cells. After 2 and 3 weeks, a significant increase in cell count was visible in both combined approaches compared to untreated cells. Thus, the addition of the combination approaches had a strong influence on the proliferation of the cells.

It can therefore be concluded that none of the additives had a cytotoxic influence during the cell count after trypsinizing the cells. Rather, it became clear that diosgenin, ricinoleic acid and the inventive examples, in particular, had a positive influence on the cell count. Especially, IE 1c provided the best result compared to Ara 1.c, Dios 1.c and Rici 1.c after both 2 and 3 weeks. (FIG 7)

### 6. Immunofluorescent Staining of the HFDPC:

Throughout the experiment, cells were stained with vimentin at defined points in time. This staining was used to prove that the cells were the declared cells during the experiment and that no differentiation of the cells or contamination of the cells occurred.

Immunofluorescence staining is used to label specific proteins with antibodies and make them visible by means of a fluorophore-coupled secondary antibody. The immunocytochemical staining was performed on sterile collagen-coated glass slides.

Vimentin is a type 3 intermediate filament found in the cytoskeleton of mesenchymal stem cells. By detecting vimentin on the cell surface, cell differentiation and contamination of the cell culture by foreign cells (assessment of these due to altered morphology) can be ruled out. The cells of the different treatment times show no differences in the vimentin staining. The secondary antibody shows no unspecific binding with other substances (FIG 8 and ,b). Thus, no degeneration of the cells occurred during the experiment and no contamination of the cell culture by other cells occurred. At the same time, it was shown that after addition of IE 1c and 2c and after addition of Dios 2c and Ara 2c, the cells were able to clearly spread and attach to the cell culture substrate. This leads to an increased cell adhesion, but is also determined by the number of cells in the respective culture well.

In the following pictures only the respective culture medium with and without added active agent is mentioned. The cells are naturally contained in each well. (FIG 9)

### 7. Cell impedance test on proliferation and toxicity

The investigation of cell proliferation (cell index CI) or the exact determination of the cell number was carried out in order to detect a possible cytotoxic effect of the substances after addition to the cells. At the beginning of the experiment, the cells were confluently seeded in order to be able to " focus " on the induction of hair growth afterwards.

The influence of various substances on the growth of HDPFC was investigated with the aid of a cell impedance device (xCELLigence® RTCA DP).

The xCELLigence® system consists of a measuring unit and software that allows to follow the cell invasion and migration in real time without the influence of additional substances for cell visualization, which could potentially have an impact on the cells. The system measures the electrical impedance via microelectrodes integrated on the bottom of the tissue culture E-plate. It enables the monitoring of changes in adhesion, spreading and proliferation of different cell types in real time, based on the measured cell-electrode impedance. From these data the socalled "Cell Index (CI)" can be calculated. This index is composed as follows:

$$CI = \frac{R_{tn} - R_{t0}}{F_i}$$

Rtn and Rt0 are the frequency-dependent electrode resistances (a component of the impedance), where Rt0 is measured at time T0 (without cells) and Rtn with cells present. Fi depends on the frequency used in the measurement.

Thus CI is a quantitative measure of the number of cells in an electrode-containing well, when comparing the same cell types. Under the same physiological conditions, more cells adhered to the electrodes result in a higher Rtn value, which leads to a higher value for CI.

In the absence of living cells (cell culture medium only) or in a suspension of dead cells, the cell index values are close to zero. After cellular attachment to the electrode, the measurement signal correlates linearly with the cell count throughout the experiment. This investigation was performed on a 96-well scale.

To perform this study 5000 HFDPC (Passage: 5) per well were seeded in the 96-well wells, coated on 50 μL collagen with the addition of a maximum of 200 μl medium. The cells were grown for 96 h in pure cell culture medium. After 96 h the "pure" cell culture medium was completely removed and medium with the respective additives was added. A further change with the respective additives took place after 48 h.

The CI is thus a measure of cell proliferation, cell morphology and cell vitality (only vital, adherent HFDPC can proliferate). (FIG 11)

A concentration of 250 μg/mL ricinoleic acid in medium does not appear to have a toxic effect on the cells in the first 24 hours after addition, but has a positive influence on the cell index. After 48h of repeated addition of 250 μg/mL ricinoleic acid the cell index decreases. The cell index at 100μg/mL ricinoleic acid remained constant after 24 h and 48 h addition.

A concentration of 50 μg/mL arachidonate in medium does not have a toxic effect on the cells, but increase the cell index. 100μg/mL arachidonate in medium has a toxic effect on the cells.

For diosgenin the measured maximum concentration of 500 μg/mL in the cellular impedance test is not toxic.

**General Preparation of Product Compositons**

All used bottles and water were sterilized (Laboclave 25MV, SHP Sterile Technique,) and all used glassware and spatulas were disinfected in a drying cabinet for 3h at 180°C.

Preparation of the diosgenin solution:

0.25 g extract of yam and 12.5 g sterile water were weighed into a Schott bottle and shaken overnight. Solution is filtered through 0.45 μm syringe filters before addition.

Preparation of Product Composition A:

**A) Preparation of the water phase:**

Montanov 202 and 85g sterile water are dissolved in a stirrer (Thermomix® TM6, Vorwerk) at 80°C. First fill in the water, heat it up, then add the emulsifier while stirring (without butterfly form stirrer).

Stir the mixture for 10 minutes, then set the temperature to 45°C.

At about 50°C, add the remaining ingredients in turn, including the water. A small amount of remaining water was used for rinsing when the oil phase is added to the water phase.

Let the water phase stir at 45°C.

**B) Preparation of the oil phase:**

Lecithin and almond oil are weighed into a 250 ml beaker (tall form) and heated to 80°C in a water bath, stirring with IKA Eurostar 6000 (about 600-700 rpm).

During the cooling phase to 50°C, the production of the water phase is continued.

When the oil phase has reached about 50°C, the remaining components are added (castor oil and phenoxyethanol were weighed directly into the beaker with the almond oil/lecithin mixture)

When the phases are well mixed, the oil phase is transferred from the beaker to the stirrer. The oil phase is added at high stirring speed. After rapid addition of the oil phase and rinsing with the remaining water, the emulsion is stirred for 10 minutes.

Dexpanthenol is then added and stirred for 10 minutes. Then the stirring attachment is changed to the butterfly form stirrer, the stirring speed is reduced until the mixture has cooled down to room temperature (about 60-70 min). Then the emulsion is filled into a 250 ml Schott bottle and shaken gently (orbital shaker) for about 1h.

The other compositions were prepared analogously.

Table 2: Product Composition **A** comprising inventive composition

| | Product composition A | | Product composition B | | Product composition C | |
|---|---|---|---|---|---|---|
| | m in [g] | [wt.%] based on 100 wt.% | m in [g] | [wt.%] based on 100 wt.% | m in [g] | [wt.%] based on 100 wt.% |
| **Water Phase:** | | | | | | |
| Water | 137 | 54.8 | 137 | 54.8 | 137 | 54.8 |
| Montanov 202 | 4.79 | 1.92 | 7.5 | 3 | 4.76 | 1.91 |
| Spectrastat G2-N | 2.89 | 1.16 | 2.88 | 1.16 | 2.88 | 1.15 |
| Potassium Sorbate | 0.5 | 0.2 | 2 | 0.8 | 0.5 | 0.2 |
| Sodium Benzoate | 0.37 | 0.15 | 1.5 | 0.6 | 0.37 | 0.15 |
| Diosgenin | 0.25 | 0.1 | 0.25 | 0.1 | 0.25 | 0.1 |
| Sodium EDTA | 0.13 | 0.05 | 0.25 | 0.1 | 0.25 | 0.1 |
| Benzalkonium Chloride | -- | -- | 0.23 | 0.09 | -- | -- |
| **Oil Phase:** | | | | | | |
| Almond Oil | 30.29 | 12.11 | 28.05 | 11.22 | 29.88 | 11.95 |
| Lecithin | 6 | 2.4 | 6 | 2.4 | 5.99 | 2.4 |
| Castor Oil | 60.25 | 24.1 | 56.3 | 22.52 | 60.1 | 24.04 |
| 2-Phenoxyethanol | 2.39 | 0.96 | 2.39 | 0.96 | 2.38 | 0.95 |
| Ricinoleic Acid | 0.25 | 0.1 | 0.25 | 0.1 | 0.25 | 0.1 |
| Sodium Arachidonate | 0.25 | 0.1 | 0.25 | 0.1 | 0.25 | 0.1 |
| Methylparaben | 0.5 | 0.2 | 1 | 0.4 | 1 | 0.4 |
| **Add to the Prepared Emulsion** | | | | | | |
| Dexpanthenol | 4.15 | 1.66 | 4.15 | 1.66 | 4.15 | 1.66 |
| Emulsion Property 1 h after Preparation | Stable | | Stable | | Stable | |
| Emulsion Property 24 h after Preparation | Stable | | Stable | | Stable | |
| Emulsion Property 1 months Preparation | Stable | | Stable | | Stable | |

| | Comperative Product composition X | | Comperative Product composition Y | | Product composition PO 500 | |
|---|---|---|---|---|---|---|
| | m in [g] | [wt.%] based on 100 wt.% | m in [g] | [wt.%] based on 100 wt.% | m in [g] | [wt.%] based on 100 wt.% |
| **Water Phase:** | | | | | | |
| Water | 37.5 | 30 | 30 | 24 | 117.23 | 46.89 |
| Montanov 202 | 3.75 | 3 | | | 7.50 | 3.00 |
| Spectrastat G2-N | 1.445 | 1.16 | 0.5 | 0.4 | 2.88 | 1.16 |
| Potassium Sorbate | 1 | 0.8 | 0.375 | 0.3 | 2.00 | 0.80 |
| Sodium Benzoate | 0.75 | 0.6 | 0.125 | 0.1 | 1.50 | 0.60 |
| Diosgenin | 0.125 | 0.1 | 0.0625 | 0.05 | 0.25 | 0.10 |
| Sodium EDTA | 0.0625 | 0.05 | 0.125 | 0.1 | 0.25 | 0.10 |
| Propylene glycol | 10 | 8 | | | | |
| Dermofeel PGPR | | | | | | |
| PO 500* | | | | | 20 | 8 |
| **Oil Phase:** | | | | | | |
| Almond Oil | 21.2 | 17.02 | 25.94 | 20.75 | 28.05 | 11.22 |
| Lecithin | 3.04 | 2.4 | 4 | 3.2 | 6.00 | 2.4 |
| Castor Oil | 42.68 | 34.11 | 52.47 | 41.98 | 56.30 | 22.52 |
| 2-Phenoxyethanol | 1 | 0.8 | 1 | 0.8 | 2.39 | 0.96 |
| Ricinoleic Acid | 0.125 | 0.1 | 0.125 | 0.1 | 0.25 | 0.10 |
| Sodium Arachidonate | 0.25 | 0.2 | | | 0.25 | 0.10 |
| Methylparaben | 21.2 | 17.02 | 0.5 | 0.4 | 1.00 | 0.40 |
| **Add to the Prepared Emulsion** | | | | | | |
| Dexpanthenol | 4.15 | 1.66 | 4.15 | 1.66 | 4.15 | 1.66 |
| Emulsion Property 1 h after Preparation | Unstable; phase separation | | Stable | | Stable | |
| Emulsion Property 24 h after Preparation | n. d. | | Unstable; phase separation | | Stable | |
| Emulsion Property 1 months Preparation | n. d. | | n. d. | | Stable | |

* PO 500 was produced by by Beauté By Roquette and contains isosorbid.

**Preservation efficacy test (DIN EN ISO 11930)**

For Product composition B a preservation efficacy test (DIN EN ISO 11930) has been undertaken.

Table 3 shows the development of the number of microbes at 20-25°C after inoculation of the sample with the described microorganisms. Table 4 shows the criteria for evaluation of antimicrobial activity as a log reduction according to DIN EN ISO 11930, in which a difference of 0.5 log units is allowed.

Product composition B satisfies the criteria A + B for preservation efficacy test according to DIN EN ISO 11930.

Table 3: Preservative efficacy test according to DIN EN ISO 11930

| **Analysis** (testing plan: B200) | **0 h** | **7 d** | **14 d** | **28 d** |
|---|---|---|---|---|
| Pseudomonas aeruginosa ATCC 9027 | $1,6 \times 10^5$ | $< 1,0 \times 10^1$ | $< 1,0 \times 10^1$ | $< 1,0 \times 10^1$ |
| Log reduction | --- | > 4,20 | > 4,20 | > 4,20 |
| Staphylococcus aureus ATCC 6538 | $2,2 \times 10^5$ | $< 1,0 \times 10^1$ | $< 1,0 \times 10^1$ | $< 1,0 \times 10^1$ |
| Log reduction | --- | > 4,34 | > 4,34 | > 4,34 |
| Escherichia coli ATCC 8739 | $3,2 \times 10^5$ | < 1,0 × 10’ | $< 1,0 \times 10^1$ | $< 1,0 \times 10^1$ |
| Log reduction | --- | > 4,51 | > 4,51 | > 4,51 |
| Candida albicans ATCC 10231 | $1,4 \times 10^4$ | $< 1,0 \times 10^1$ | $< 1,0 \times 10^1$ | $< 1,0 \times 10^1$ |
| Log reduction | --- | > 3,15 | > 3,15 | > 3,15 |
| Aspergillus brasiliensis ATCC 16404 | $3,9 \times 10^4$ | --- | $< 1,0 \times 10^1$ | $< 1,0 \times 10^1$ |
| Log reduction | --- | --- | > 3,59 | > 3,59 |
| all counts in cfu/g or ml (clf = colony forming units) | | | | |

Table 4: Criteria for evaluation of antimicrobial activity

| | **0 h** | **7 d** | **14 d** | **28 d** |
|---|---|---|---|---|
| Bacteria (Criteria A) | --- | ≥ 3,0 | ≥ 3,0 * | ≥ 3,0 * |
| Bacteria (Criteria B) | --- | --- | ≥ 3,0 | ≥ 3,0 * |
| Candida albicans (Criteria A) | --- | ≥ 1,0 | ≥ 1,0 * | ≥ 1,0 * |
| Candida albicans (Criteria B) | --- | --- | ≥ 1,0 | ≥ 1,0 * |
| Aspergillus brasiliensis (Criteria A) | --- | --- | ≥ 0,0 | ≥ 1,0 * |
| Aspergillus brasiliensis (Criteria B) | --- | --- | ≥ 0,0 | ≥ 0,0* |
| * no increase since last count | | | | |

**Application of the inventive composition (product composition)**

A product composition comprising the inventive composition has been tested by a female human test person for 5 weeks.

At the beginning, the eyelash length of the test person was 4 mm, measured with a ruler. After daily using the product composition for 5 weeks on the right eyelash the right eyelash length of the test person increased to 8 mm, measured with a ruler and photographed with a digital camera (FIG 12, (B)).The left eyelash (FIG 12, (A)) which was not treated with the product composition, remained at 4 mm after 5 weeks as to the beginning, as it was also measured with a ruler and photographed with a digital camera. Therefore, the right eyelash length is 100% longer than in the beginning. Additionally, the right eyelashes became thicker as can be gathered from the photos.

**Claims**

1. Composition comprising

    a. 0.01 wt.% - 2.0 wt.%, preferably 0.05 wt.% - 0.5 wt.%, ricinoleic acid and/ or its salt based on the total weight of the composition, and
    b. 0.01 wt.% - 2.0 wt.%, preferably 0.05 wt.% - 0.5 wt.%, arachidonate based on the total weight of the composition.

2. Composition according to claim 1, wherein the composition comprises 0.01 wt.% - 2.0 wt.%, preferably 0.05 wt.% - 0.5 wt.% diosgenin based on the total weight of the composition.

**3.** Composition comprising

a. 0.01 wt.% - 2.0 wt.%, preferably 0.05 wt.% - 0.5 wt.% ricinoleic acid and/ or its salt based on the total weight of the composition, and
b. 0.01 wt.% - 2.0 wt.%, preferably 0.05 wt.% - 0.5 wt.% diosgenin based on the total weight of the composition.

**4.** Composition according to claim 3, wherein the composition comprises 0.01 wt.% - 2.0 wt.%, preferably 0.05 wt.% - 0.5 wt.%, arachidonate based on the total weight of the composition.

**5.** Composition according to one of the preceding claims, wherein the composition comprises a concentrated secretome of totipotent cells from *Curcuma longa* rhizome.

**6.** Composition according to one of the preceding claims, wherein the composition does not comprise any hormones.

**7.** Composition according to one of the preceding claims, wherein the composition comprises vitamins, such as vitamin C, vitamin B1 , vitamin B3, vitamin B5, vitamin B6, vitamin B2, vitamin B8, vitamin B9, and/ or mixtures thereof.

**8.** Composition according to one of the preceding claims, wherein the composition comprises additives, such as antioxidants, emulsifiers, preservatives, colorants, UV filters, acids, bases, pH stabilizer and perfumes.

**9.** Composition according to one of the preceding claims, wherein the composition does not comprise prostaglandin A2, prostaglandin F2, prostaglandin E1, prostaglandin E2, Arbaprostil, Carboprost, Enprostil, Bimatoprost, Bemeprost, Latanaoprost, Limaprost, Misoprostol, Ornoprostil, Prostacyclin, Prostaglandin E1, Prostaglandin E2, Prostaglandin F2$\alpha$, Rioprostil, Rosaprostol, Sulprostone, Travaprost, Trimoprostil, Viprostol, 15-PGDH inhibitors.

**10.** Composition according to one of the preceding claims, wherein the composition does not comprise minoxidil and/ or finasterid.

**11.** Composition according to one of the preceding claims for use as a cosmetic.

**12.** Composition according to one of the preceding claims 1-10 for use as a medicament.

**13.** Composition according to one of the preceding claims 1-11 and 12 for use in a method for the treatment of hair loss, preferably hypotrichosis of humans.

**14.** Composition according to one of the preceding claims 1-11 and 12-13 for use in a method for the treatment of hypotrichosis and/ or alopecia of eyelashes, eyebrows and scalp hair of humans.

## Patentansprüche

**1.** Zusammensetzung enthaltend

a. 0,01 Gew.-% - 2,0 Gew.-%, vorzugsweise 0,05 Gew.-% - 0,5 Gew.-%, Ricinolsäure und/oder deren Salz, bezogen auf das Gesamtgewicht der Zusammensetzung, und
b. 0,01 Gew.-% - 2,0 Gew.-%, vorzugsweise 0,05 Gew.-% - 0,5 Gew.-%, Arachidonat, bezogen auf das Gesamtgewicht der Zusammensetzung.

**2.** Zusammensetzung nach Anspruch 1, wobei die Zusammensetzung 0,01 Gew.-% - 2,0 Gew.-%, vorzugsweise 0,05 Gew.-% - 0,5 Gew.-% Diosgenin, bezogen auf das Gesamtgewicht der Zusammensetzung, enthält.

**3.** Zusammensetzung enthaltend

a. 0,01 Gew.-% - 2,0 Gew.-%, vorzugsweise 0,05 Gew.-% - 0,5 Gew.-% Ricinolsäure und/oder deren Salz, bezogen auf das Gesamtgewicht der Zusammensetzung, und
b. 0,01 Gew.-% - 2,0 Gew.-%, vorzugsweise 0,05 Gew.-% - 0,5 Gew.-% Diosgenin, bezogen auf das Gesamtgewicht der Zusammensetzung.

4. Zusammensetzung nach Anspruch 3, wobei die Zusammensetzung 0,01 Gew.-% - 2,0 Gew.-%, vorzugsweise 0,05 Gew.-% - 0,5 Gew.-%, Arachidonat, bezogen auf das Gesamtgewicht der Zusammensetzung, enthält.

5. Zusammensetzung nach einem der vorhergehenden Ansprüche, wobei die Zusammensetzung ein konzentriertes Sekretom totipotenter Zellen aus *Curcuma longa* Rhizom enthält.

6. Zusammensetzung nach einem der vorhergehenden Ansprüche, wobei die Zusammensetzung keine Hormone enthält.

7. Zusammensetzung nach einem der vorhergehenden Ansprüche, wobei die Zusammensetzung Vitamine, wie Vitamin C, Vitamin B1, Vitamin B3, Vitamin B5, Vitamin B6, Vitamin B2, Vitamin B8, Vitamin B9 und/oder Mischungen davon, enthält.

8. Zusammensetzung nach einem der vorhergehenden Ansprüche, wobei die Zusammensetzung Zusatzstoffe, wie Antioxidantien, Emulgatoren, Konservierungsmittel, Farbstoffe, UV-Filter, Säuren, Basen, pH-Stabilisatoren und Duftstoffe, enthält.

9. Zusammensetzung nach einem der vorhergehenden Ansprüche, wobei die Zusammensetzung folgende Stoffe nicht enthält: Prostaglandin A2, Prostaglandin F2, Prostaglandin E1, Prostaglandin E2, Arbaprostil, Carboprost, Enprostil, Bimatoprost, Bemeprost, Latanaoprost, Limaprost, Misoprostol, Ornoprostil, Prostacyclin, Prostaglandin E1, Prostaglandin E2, Prostaglandin F2$\alpha$, Rioprostil, Rosaprostol, Sulprostone, Travaprost, Trimoprostil, Viprostol, 15-PGDH-Inhibitoren.

10. Zusammensetzung nach einem der vorhergehenden Ansprüche, wobei die Zusammensetzung kein Minoxidil und/oder Finasterid enthält.

11. Zusammensetzung nach einem der vorhergehenden Ansprüche zur Verwendung als Kosmetikum.

12. Zusammensetzung nach einem der vorhergehenden Ansprüche 1-10 zur Verwendung als Arzneimittel.

13. Zusammensetzung nach einem der vorhergehenden Ansprüche 1-11 und 12 zur Verwendung in einem Verfahren zur Behandlung von Haarausfall, vorzugsweise von Hypotrichose bei Menschen.

14. Zusammensetzung nach einem der vorhergehenden Ansprüche 1-11 und 12-13 zur Verwendung in einem Verfahren zur Behandlung von Hypotrichose und/oder Alopezie der Wimpern, Augenbrauen und Kopfhaare bei Menschen.

## Revendications

1. Composition comprenant

   a. de 0,01 % en poids à 2,0 % en poids, de préférence de 0,05 % en poids à 0,5 % en poids, d'acide ricinoléique et/ou de son sel par rapport au poids total de la composition, et
   b. de 0,01 % en poids à 2,0 % en poids, de préférence de 0,05 % en poids à 0,5 % en poids, d'arachidonate par rapport au poids total de la composition.

2. Composition selon la revendication 1, dans laquelle la composition comprend de 0,01 % en poids à 2,0 % en poids, de préférence de 0,05 % en poids à 0,5 % en poids, de diosgénine par rapport au poids total de la composition.

3. Composition comprenant

   a. de 0,01 % en poids à 2,0 % en poids, de préférence de 0,05 % en poids à 0,5 % en poids d'acide ricinoléique et/ou de son sel par rapport au poids total de la composition, et
   b. de 0,01 % en poids à 2,0 % en poids, de préférence de 0,05 % en poids à 0,5 % en poids, de diosgénine par rapport au poids total de la composition.

4. Composition selon la revendication 3, dans laquelle la composition comprend de 0,01 % en poids à 2,0 % en poids, de préférence de 0,05 % en poids à 0,5 % en poids, d'arachidonate par rapport au poids total de la composition.

**5.** Composition selon l'une des revendications précédentes, dans laquelle la composition comprend un sécrétome concentré de cellules totipotentes issues du rhizome de *Curcuma longa.*

**6.** Composition selon l'une des revendications précédentes, dans laquelle la composition ne comprend pas d'hormones.

**7.** Composition selon l'une des revendications précédentes, dans laquelle la composition comprend des vitamines, telles que vitamine C, vitamine B1, vitamine B3, vitamine B5, vitamine B6, vitamine B2, vitamine B8, vitamine B9 et/ou leurs mélanges.

**8.** Composition selon l'une des revendications précédentes, dans laquelle la composition comprend des additifs, tels qu'antioxydants, émulsifiants, conservateurs, colorants, filtres UV, acides, bases, stabilisateurs de pH et parfums.

**9.** Composition selon l'une des revendications précédentes, dans laquelle la composition ne comprend pas de prostaglandine A2, prostaglandine F2, prostaglandine E1, prostaglandine E2, arbaprostil, carboprost, enprostil, bimatoprost, bemeprost, latanaoprost, limaprost, misoprostol, ornoprostyl, prostacycline, prostaglandine E1, prostaglandine E2, prostaglandine F2$\alpha$, rioprostil, rosaprostol, sulprostone, travaprost, trimoprostil, viprostol, inhibiteurs de 15-PGDH.

**10.** Composition selon l'une des revendications précédentes, dans laquelle la composition ne comprend pas de minoxidil et/ou de finastéride.

**11.** Composition selon l'une des revendications précédentes pour une utilisation en tant que cosmétique.

**12.** Composition selon l'une des revendications 1 à 10 précédentes pour une utilisation en tant que médicament.

**13.** Composition selon l'une des revendications 1 à 11 et 12 précédentes pour une utilisation dans un procédé de traitement de la perte de cheveux, de préférence de l'hypotrichose chez l'être humain.

**14.** Composition selon l'une des revendications 1 à 11 et 12 à 13 précédentes pour une utilisation dans un procédé de traitement de l'hypotrichose et/ou de l'alopécie des cils, des sourcils et du cuir chevelu chez l'être humain.

FIG 1

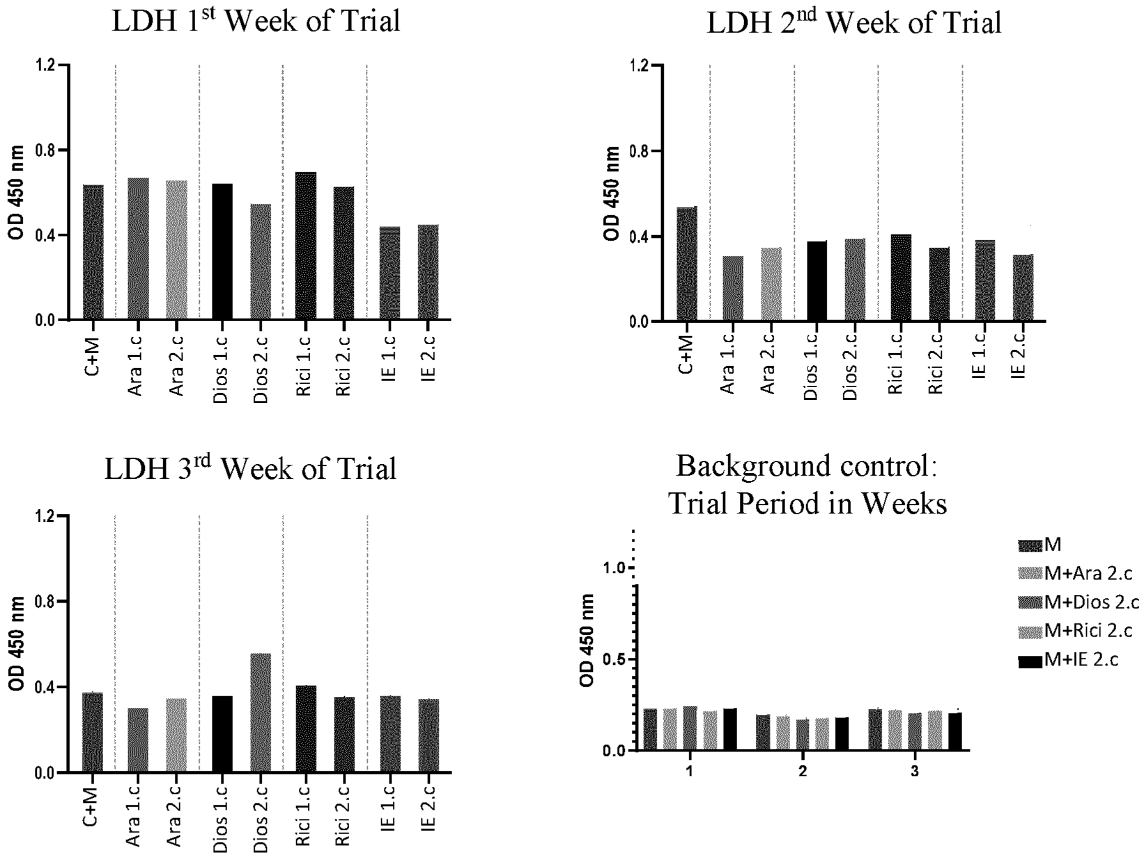
LDH 1st Week of Trial
LDH 2nd Week of Trial
LDH 3rd Week of Trial
Background control:
Trial Period in Weeks
OD 450 nm
1.2
0.8
0.4
0.0
1.0
0.5
C+M
Ara 1.c
Ara 2.c
Dios 1.c
Dios 2.c
Rici 1.c
Rici 2.c
IE 1.c
IE 2.c
1
2
3
M
M+Ara 2.c
M+Dios 2.c
M+Rici 2.c
M+IE 2.c

**FIG 2**

a) 10x magnification, b) 20x magnification

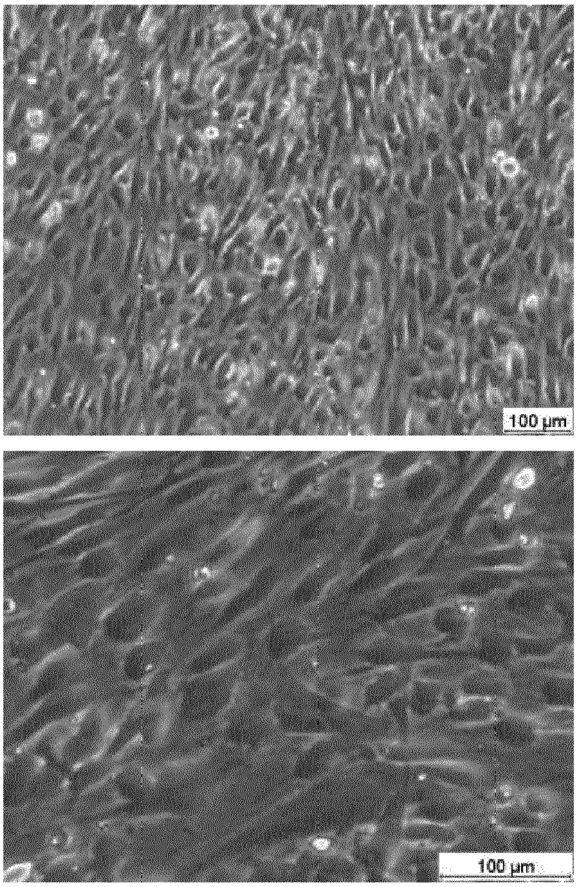

**FIG 3**

a) Ara 1.c, b) Ara 2.c, c) Rici 1.c, d) Rici 3.c, e) Diosgenin 1.c, f) Diosgenin 2.c, g) IE 1.c, h) IE 2.c

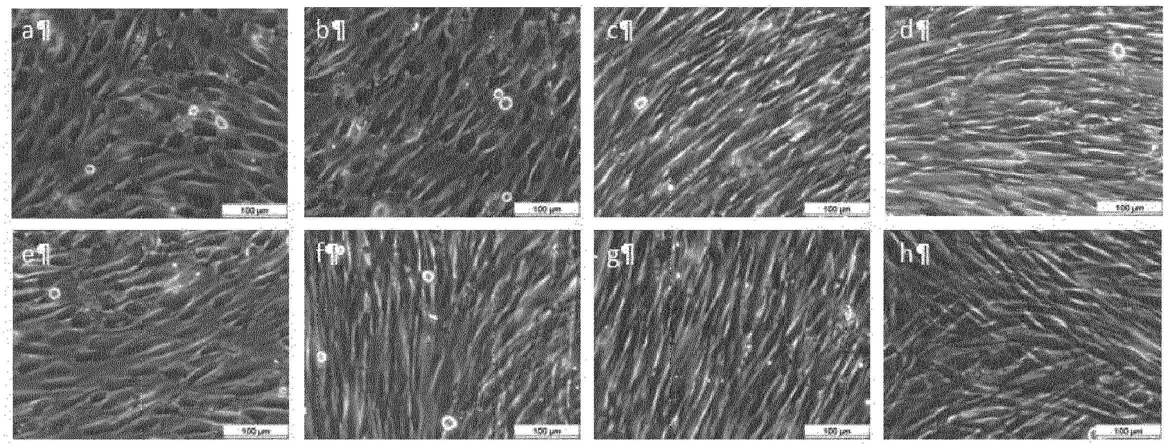

**FIG 4**

a) Medium b) Ara 1.c, c) Ara 2.c, d) Rici 1.c, e) Rici 3.c, f) Diosgenin 1.c, g) Diosgenin 2.c, h) IE 1.c, i) IE 2.c

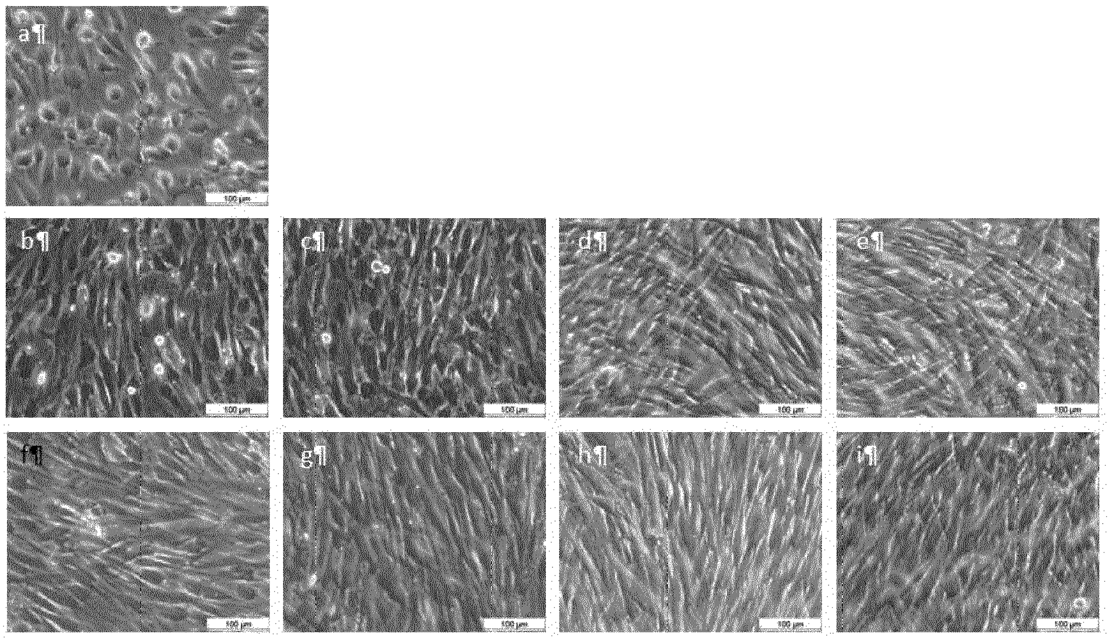

## FIG 5

a) Medium b) Ara 1.c, c) Ara 2.c, d) Rici 1.c, e) Rici 3.c, f) Diosgenin 1.c, g) Diosgenin 2.c, h) IE 1.c, i) IE 2.c

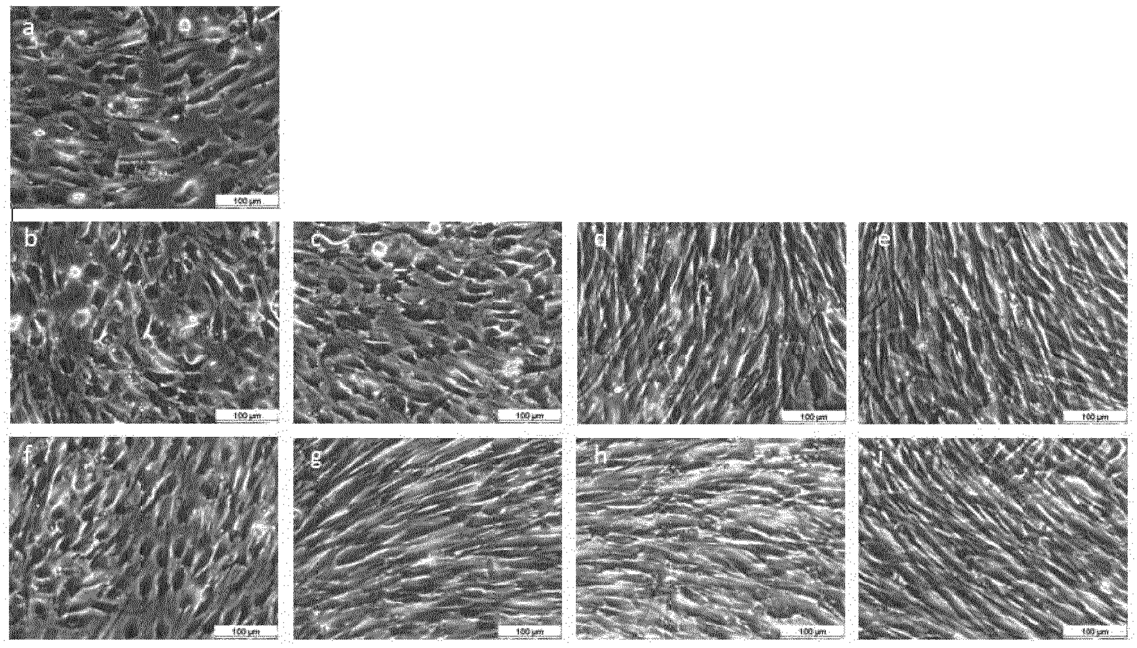

FIG 6

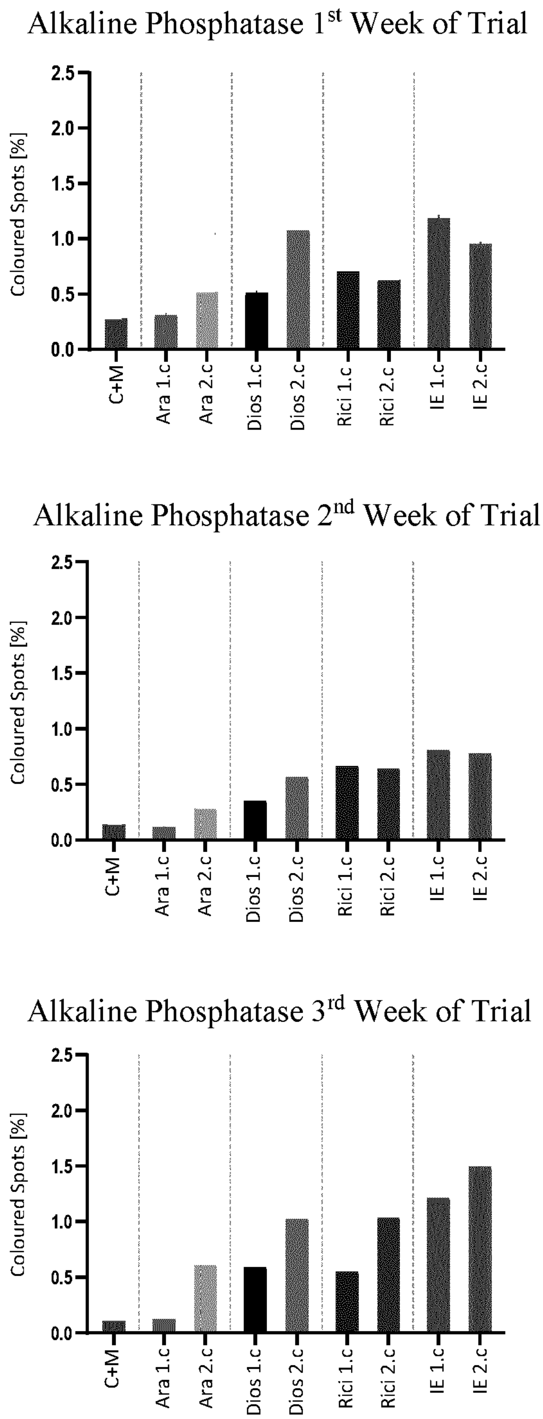
Alkaline Phosphatase 1st Week of Trial
Alkaline Phosphatase 2nd Week of Trial
Alkaline Phosphatase 3rd Week of Trial
Coloured Spots [%]
0.0
0.5
1.0
1.5
2.0
2.5
C+M
Ara 1.c
Ara 2.c
Dios 1.c
Dios 2.c
Rici 1.c
Rici 2.c
IE 1.c
IE 2.c

FIG 7

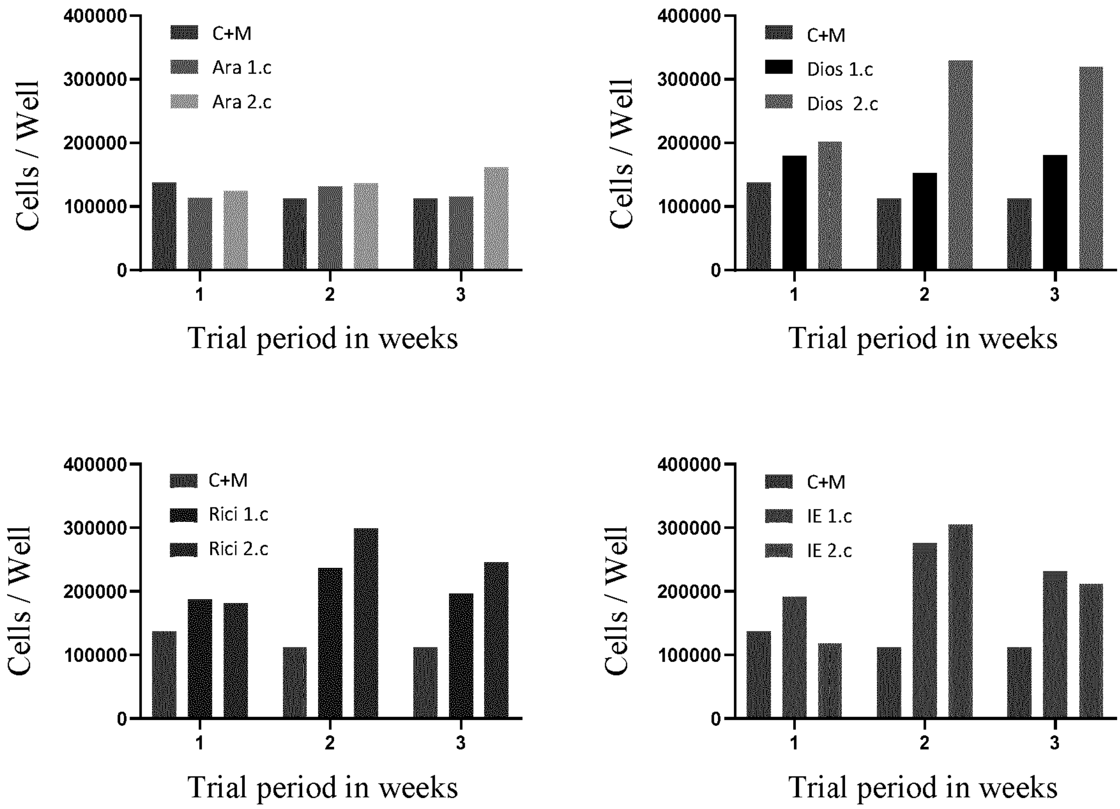
400000
300000
200000
100000
0
Cells / Well
C+M
Ara 1.c
Ara 2.c
1
2
3
Trial period in weeks
C+M
Dios 1.c
Dios 2.c
Rici 1.c
Rici 2.c
IE 1.c
IE 2.c

**FIG 8**

Cells were fixed with formaldehyde and labeled with anti vimentin/anti rabbit AlexaFluor 555 (red).

a) medium, b) medium without primary antibodies, c) Ara 1.c, d) Ara 2.c, e) Rici 1.c, f) Rici 2.c, g) Diosgenin 1.c, h) Diosgenin 2.c, i) IE 1.c, j) IE 2.c, k) DFMO 1.c, l) DFMO 2.c

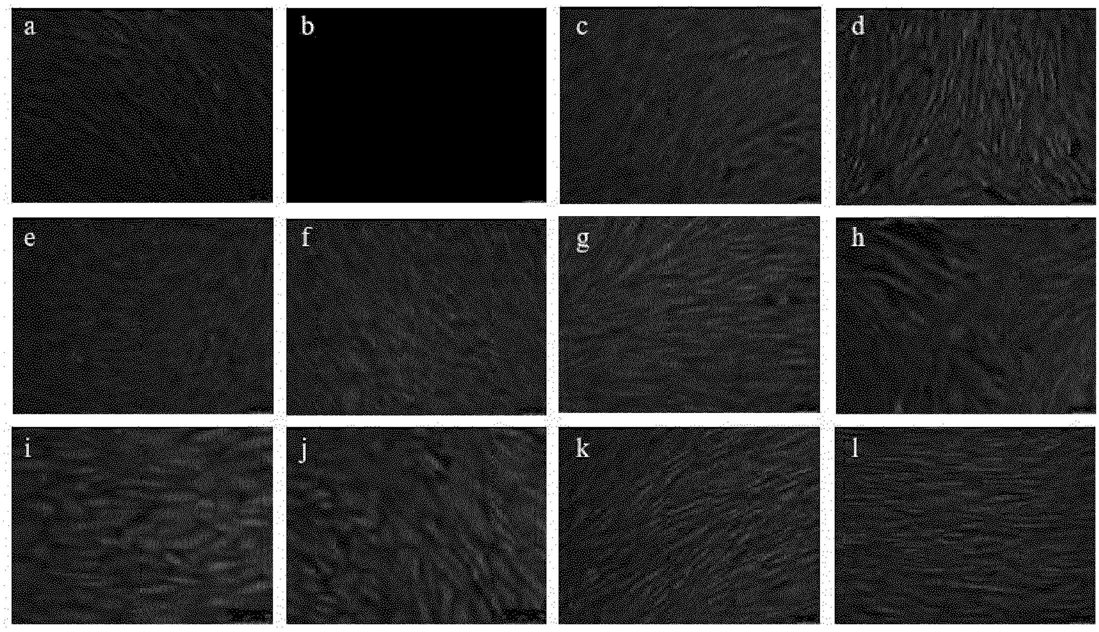

**FIG 9**

Cells were fixed with formaldehyde and labeled with anti vimentin/anti rabbit AlexaFluor 555 (red). The cell core staining was done with DAPI (blue).

a) medium, b) medium without primary antibodies, c) Ara 1.c, d) Ara 2.c, e) Rici 1.c, f) Rici 2.c, g) Dios 1.c, h) Dios 2.c, i) IE 1.c, j) IE 2.c, k) DFMO 1.c, l) DFMO 2.c

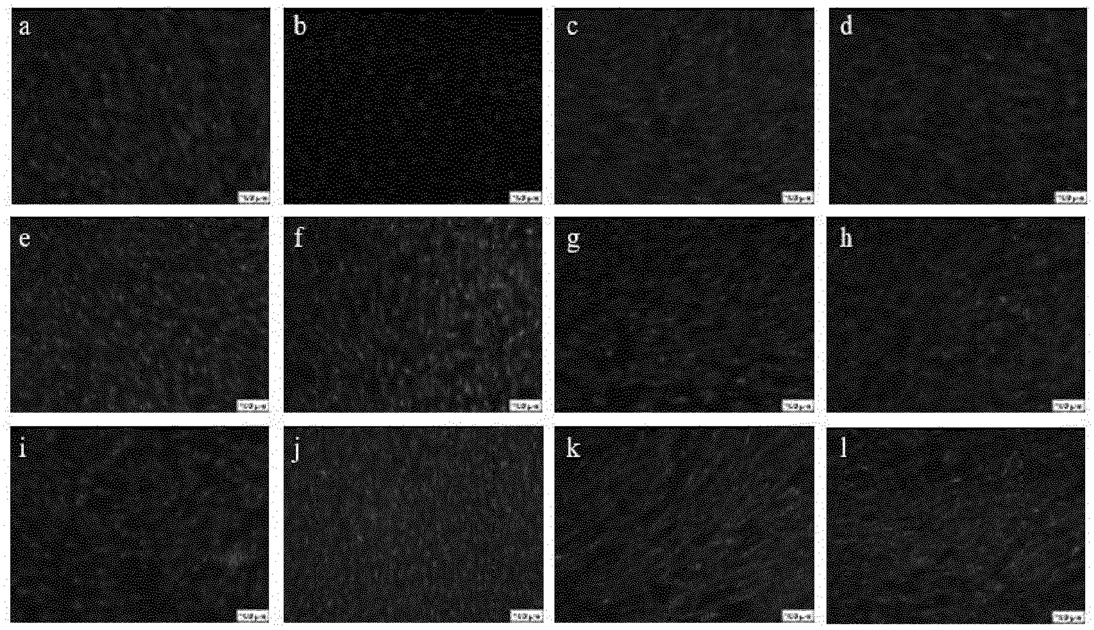

FIG 10

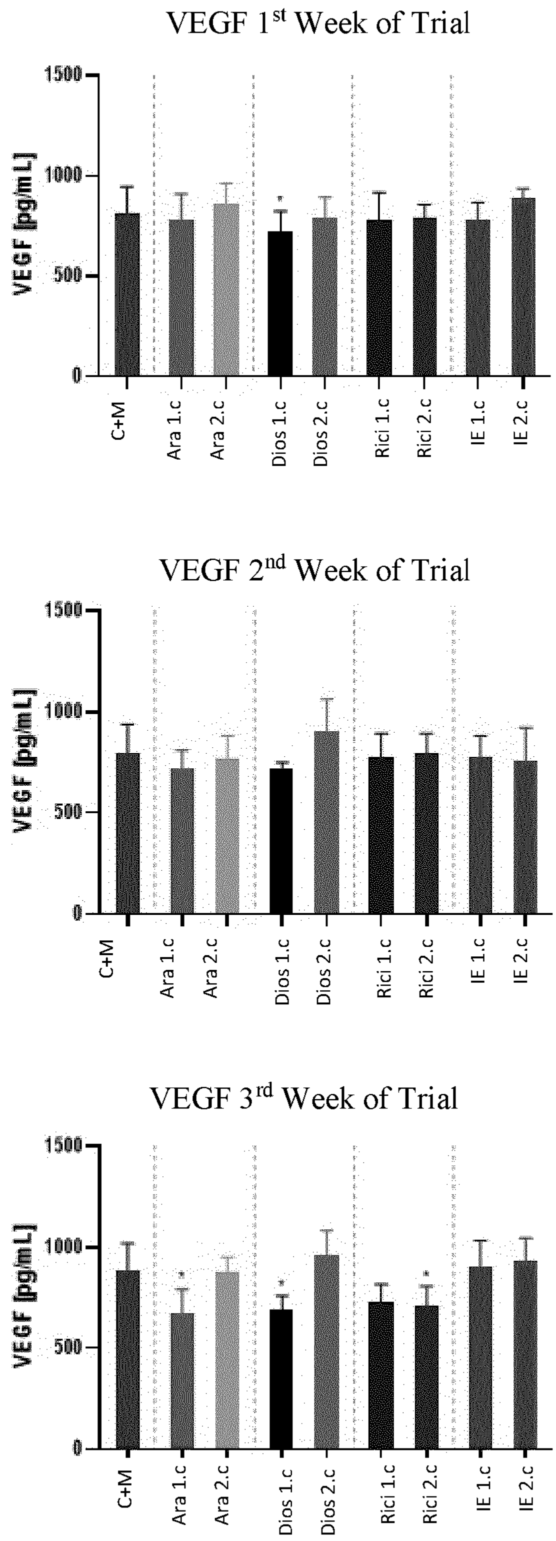
VEGF 1st Week of Trial
VEGF [pg/mL]
1500
1000
500
0
C+M
Ara 1.c
Ara 2.c
Dios 1.c
Dios 2.c
Rici 1.c
Rici 2.c
IE 1.c
IE 2.c
VEGF 2nd Week of Trial
VEGF [pg/mL]
1500
1000
500
0
C+M
Ara 1.c
Ara 2.c
Dios 1.c
Dios 2.c
Rici 1.c
Rici 2.c
IE 1.c
IE 2.c
VEGF 3rd Week of Trial
VEGF [pg/mL]
1500
1000
500
0
C+M
Ara 1.c
Ara 2.c
Dios 1.c
Dios 2.c
Rici 1.c
Rici 2.c
IE 1.c
IE 2.c

**FIG 11**

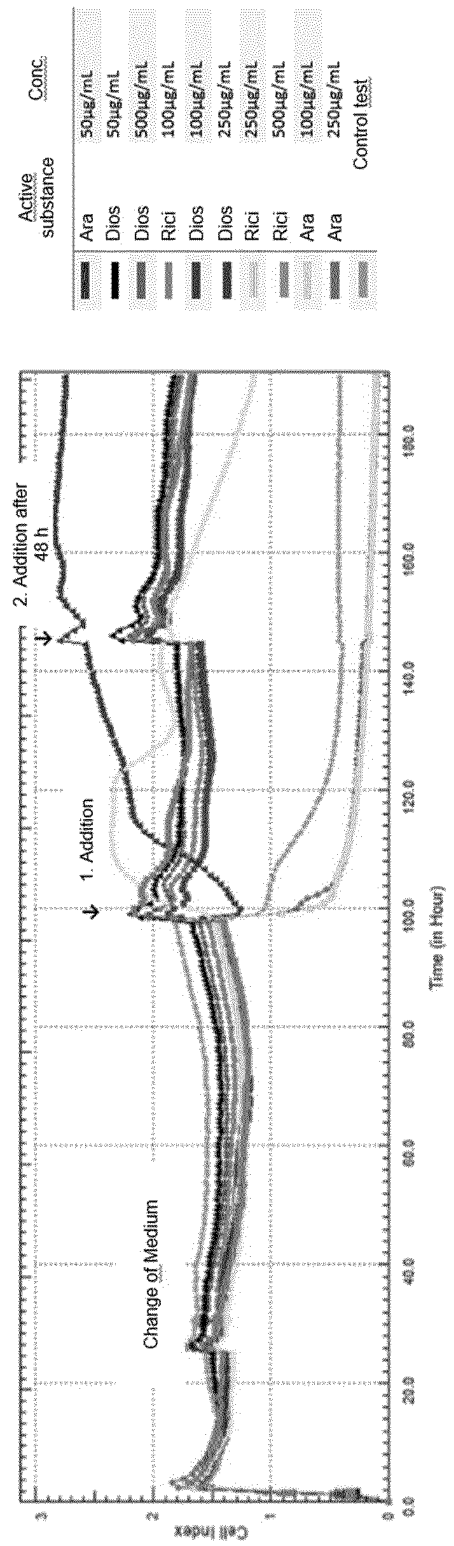
Active substance
Conc.
Ara 50µg/mL
Dios 50µg/mL
Dios 500µg/mL
Rici 100µg/mL
Dios 100µg/mL
Dios 250µg/mL
Rici 250µg/mL
Rici 500µg/mL
Ara 100µg/mL
Ara 250µg/mL
Control test
2. Addition after 48 h
1. Addition
Change of Medium
Cell Index
Time (in Hour)

FIG 12

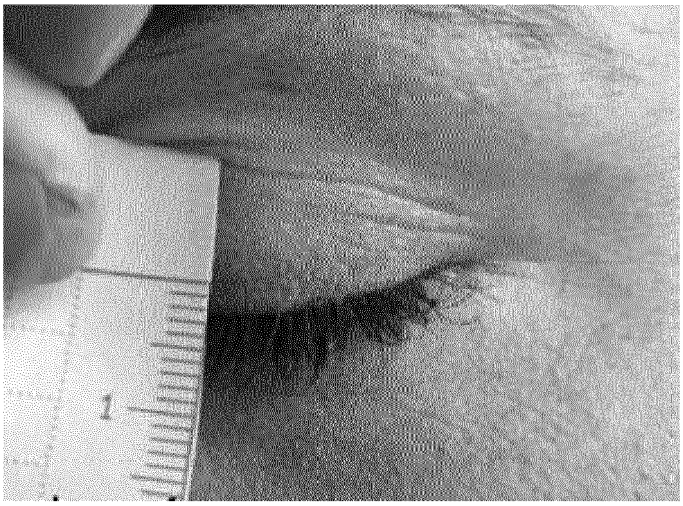

(A)

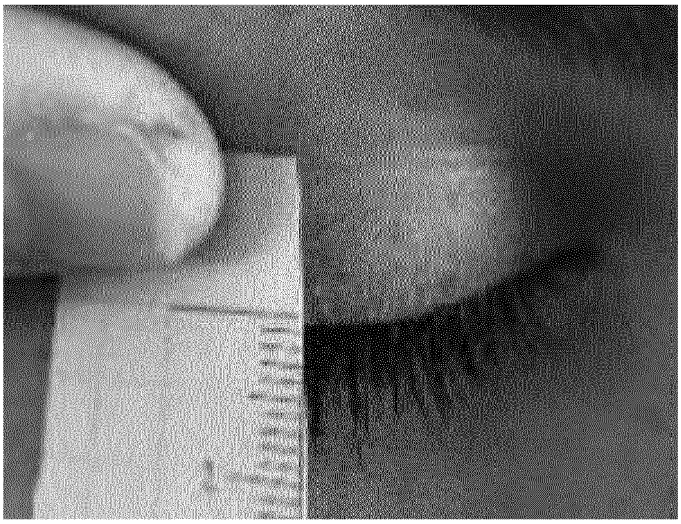

(B)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4139619 A
- US 4596812 A
- EP 0353123 A
- EP 0356271 A
- EP 0408442 A
- EP 0522964 A
- EP 0420707 A
- EP 0459890 A
- EP 0519819 A
- WO 9833497 A
- US 6262105 B
- US 20080275118 A1
- US 5827510 A
- JP S59152307 A
- JP 2006273754 A
- WO 2017178250 A1


### Non-patent literature cited in the description


- **MURRAY A.** ; **JOHNSTONE M. D.** *Am. J. Opht.*, 1997, vol. 124 (4), 544-547
- **ROENIGK H. H.** *Clinic Dermatol*, 1988, vol. 6 (4), 119-121
- **WOLF et al.** *Dermatology Online Journal*, 2003, vol. 9 (3), 7
- **FONG et al.** *Journal of Ethnopharmacology*, 2015, vol. 175, 470
- **SEMCHIN et al.** *Annals of Dermatology*, 2016, vol. 28 (1), 55